# EUROPÄISCHE PATENTSCHRIFT

(11) **EP 3 614 977 B1**
(45) Veröffentlichungstag und Bekanntmachung des Hinweises auf die Patenterteilung: **02.06.2021**
(21) Anmeldenummer: 18720585.1
(22) Anmeldetag: 25.04.2018
(51) Int. Cl.: A61F 2/78, A61F 2/80

(54) **LINERSYSTEM UND VERFAHREN ZUM ANLEGEN EINES LINERSYSTEMS**
LINER SYSTEM AND METHOD FOR APPLYING A LINER SYSTEM
SYSTÈME DE REVÊTEMENT ET PROCÉDÉ SERVANT À POSER UN SYSTÈME DE REVÊTEMENT

(30) Priorität: 26.04.2017 DE 102017108913
(43) Veröffentlichungstag der Anmeldung: 04.03.2020
(73) Patentinhaber: Ottobock SE & Co. KGaA, 37115 Duderstadt (DE)
(72) Erfinder: MÜLLER, Christian, 37589 Kalefeld (DE); BIELEFELD, Thilo-Mathias, 29416 Kuhfelde (DE)
(74) Vertreter: Gramm, Lins & Partner Patent- und Rechtsanwälte PartGmbB
(86) Internationale Anmeldenummer: PCT/EP2018/060624
(87) Internationale Veröffentlichungsnummer: WO 2018/197574

(56) Entgegenhaltungen:
- WO-A1-2013/028647
- DE-A1- 10 142 491
- DE-T2- 69 826 917

## Beschreibung

Die Erfindung betrifft ein Linersystem zur Anlage an einer Gliedmaße oder an einen Gliedmaßenstumpf, mit einem Innenliner, mit einer der Gliedmaße oder dem Gliedmaßenstumpf zugewandten Innenseite und einer der Gliedmaße oder dem Gliedmaßenstumpf abgewandten Außenseite sowie mit einem Außenliner, der eine dem Innenliner zugewandten Innenseite und eine dem Innenliner abgewandten Außenseite aufweist und zur Anlage über den Innenliner ausgebildet, wie in den Ansprüchen definiert. Die Erfindung betrifft ebenfalls ein Verfahren zum Anlegen eines solchen Linersystems, wie in den Ansprüchen definiert. Ein solches Linersystem ist insbesondere zur Verwendung als Prothesenlinersystem vorgesehen, um eine Prothesenkomponente, insbesondere einen Prothesenschaft, an einem Gliedmaßenstumpf anzulegen. Grundsätzlich besteht die Möglichkeit, dass auch Orthesen über ein Linersystem an der Gliedmaße festgelegt werden. Auch hier besteht Bedarf an einer erhöhten Individualisierbarkeit bei einfacher Produktion.

Prothesen sollen die Funktion und gegebenenfalls die Form einer nicht vorhandenen oder nicht mehr vorhandenen Gliedmaße ersetzen. Dazu ist es notwendig, dass die Prothese an der noch vorhandenen Gliedmaße oder an dem Körper des Prothesennutzers festgelegt wird. Hierzu existieren unterschiedliche Lösungssysteme, mit denen es möglich ist, Prothesenkomponenten, insbesondere einen Prothesenschaft, an einer Gliedmaße oder einem Gliedmaßenstumpf festzulegen. Neben einem Befestigen über Gurte oder Riemen wird heutzutage zunehmend ein Linersystem bevorzugt, bei dem ein Liner über eine Gliedmaße oder einen Gliedmaßenstumpf gezogen wird, um eine Schnittstelle oder einen Interface zwischen dem Gliedmaßenstumpf und dem Prothesenschaft, an dem weitere Prothesenkomponenten angeordnet sind, herzustellen. Der Liner bildet eine Schutzschicht für die Hautoberfläche und gleichzeitig eine Polsterung, um Druckbelastungen gleichmäßig zu verteilen. Ebenfalls schützt der Prothesenliner vor Hautirritationen. Darüber hinaus können an dem Prothesenliner mechanische Verriegelungseinrichtungen wie ein Verriegelungsstift oder ein Zuggurt befestigt sein, über den es möglich ist, den Prothesenliner fest mit der daran festzulegenden Prothesenkomponente zu verbinden.

Neben einer Verriegelung über ein sogenanntes Pinlock-System oder einen Gurt besteht die Möglichkeit, den Prothesenschaft über ein Unterdrucksystem an den Liner zu koppeln. Dazu wird der Prothesenliner über die Gliedmaße gezogen und ein abgedichtetes Volumen zwischen der Außenseite des Prothesenliners und der Innenseite des Prothesenschaftes ausgebildet, aus dem dann die Luft evakuiert wird. Durch einen Unterdruck in dem Raum zwischen der Außenseite des Prothesenliners und der Innenseite des Prothesenschaftes wird der Prothesenschaft sicher an dem Prothesenliner gehalten. Zum Lösen des Prothesenschaftes wird Luft in den Zwischenraum eingelassen. Zur Abdichtung an dem oberen Schaftrand besteht die Möglichkeit, den oberen Rand des Prothesenliners, der über den oberen Rand des Prothesenschaftes hinaussteht, umzuschlagen. Ein doppelwandiger Liner mit einer umlaufenden Verdickung auf der umgeschlagenen Außenseite eines Außenliners ist aus der US 9,192,488 B2 bekannt. Alternativ dazu besteht die Möglichkeit, auf der Außenseite des Prothesenliners eine Dichtlippe anzuordnen, um die Ausbildung eines geschlossenen Raumes zwischen der Außenseite des Prothesenliners und der Innenseite des Prothesenschaftes zu gewährleisten. Solche Linersysteme sind beispielsweise aus der WO 2004-0601136 A2 bekannt.

Die WO 2013/028647 A1 betrifft einen Prothesenliner zur Anordnung zwischen einem Stumpf und einem Prothesenschaft mit einem langgestreckten, im Wesentlichen konischen Grundkörper aus einem zumindest radial elastisch ausdehnbaren Material. Der Linergrundkörper weist proximale und distale Endbereiche auf. Eine Dichtkomponente ist mit dem Linergrundkörper verbunden und hat zumindest eine äußere Ausgestaltung entlang der äußeren Oberfläche der Dichtkomponente zum Ineingrifftreten mit einem Prothesenschaft und zumindest eine innere Ausgestaltung, um beweglich mit dem Linergrundkörper in Verbindung zu treten.

Die DE 101 42 491 B4 betrifft eine Dichtanordnung zum Abdichten eines Amputationsstumpfes in einem becherförmigen, luftdichten Prothesenschaft, mit einem auf den Amputationsstumpf aufziehbaren Träger, der den Amputationsstumpf in Umfangsrichtung allseitig umschließt. Der Träger weist zumindest eine Dichtlippe auf, die eine Wurzel und eine von der Wurzel abliegende, anschmiegsame Dichtkante aufweist, die gegen die Innenseite des Prothesenschaftes abdichtend anliegen soll, wobei im Gebrauchsstand die Dichtkante bezogen auf den Amputationsstumpf proximal und die Wurzel distal liegt. Die Dichtlippe umgibt den Amputationsstumpf in Umfangsrichtung zumindest teilweise und ist mit ihrer Wurzel an dem Träger befestigt. Die Rückseite der Dichtlippe kann zur Umgebung belüftet werden.

Die DE 10 2014 006 689 A1 betrifft einen Dichtring zur Befestigung an einem proximalen Ende einer Stumpfaufnahme, mit einem hülsenförmigen Grundkörper, der Befestigungsmittel zur Festlegung des Grundkörpers an der Stumpfaufnahme aufweist. An dem Grundkörper ist eine nach innen stehende Dichtlippe angeordnet.

Die US 8,097,043 B2 betrifft einen Prothesenliner mit einem langgestreckten, im Wesentlichen konischen Grundkörper aus einem Material, das zumindest radial dehnbar ist und ein offenes proximales und ein geschlossenes distales Ende ausbildet. Die äußerste Schicht des Grundkörpers ist von einer Textilabdichtung gebildet. Zumindest ein nachgiebiges Dichtelement aus einem Elastomermaterial erstreckt sich entlang der äußersten Schicht. Zumindest ein Dichtelement erstreckt sich nach außen von dem distalen Bereich zu dem proximalen Bereich, so dass die Dicke des zumindest einen Dichtelementes im proximalen Bereich größer als die Dicke im distalen Bereich ist. Der proximale Bereich weist einen Querschnitt auf, der sich im Wesentlichen senkrecht zu der Längserstreckung des Linergrundkörpers erstreckt. Das zumindest eine Dichtelement erstreckt sich ringförmig um den Grundkörper an dessen distalem Ende.

Die DE 698 26 917 T2 1 betrifft ein System zur Anpassung eines prothetischen Instrumentes auf einen Stumpf mit einem Innenfutter, einem Zwischenfutter und einem Außenfutter, das elastisch ausgebildet ist und außen um das Innenfutter und das Zwischenfutter gelegt wird. Gelgefüllte Kissen werden zwischen das Innenfutter und das Zwischenfutter zum Ausgleich von Unregelmäßigkeiten in der Kontur des Stumpfes eingelegt. Über das Zwischenfutter wird dann das elastische Außenfutter gezogen.

Ein Liner mit einer angeformten äußeren Dichtlippe ist vergleichsweise schwierig herzustellen. Ein um den Prothesenschaftrand umzuschlagender, doppelwandiger Liner benötigt zusätzliches Material und führt zu einer Umfangsvergrößerung im proximalen Bereich des Prothesenschaftes. Darüber hinaus ist eine Individualisierbarkeit des Prothesenliners nicht möglich.

Aufgabe der vorliegenden Erfindung ist es, ein Linersystem und ein Verfahren zum Anlegen eines Liners bereitzustellen, mit denen eine einfache Individualisierbarkeit bei einfacher und kostengünstiger Herstellung gewährleistet werden kann.

Erfindungsgemäß wird die Aufgabe durch ein Linersystem mit den Merkmalen des Hauptanspruches und ein Verfahren mit den Merkmalen des nebengeordneten Anspruches gelöst. Vorteilhafte Ausgestaltungen und Weiterbildungen der Erfindung sind in den Unteransprüchen, in der Beschreibung sowie in den Figuren offenbart.

Das Linersystem zur Anlage an einer Gliedmaße oder an einem Gliedmaßenstumpf, mit einem Innenliner, der eine der Gliedmaße oder dem Gliedmaßenstumpf zugewandte Innenseite und eine Gliedmaße oder dem Gliedmaßenstumpf abgewandte Außenseite aufweist, sowie mit einem Außenliner, mit einer dem Innenliner zugewandten Innenseite und einer dem Innenliner abgewandten Außenseite, der zur Anlage über dem Innenliner vorgesehen ist, sieht vor, dass ein separat von dem Innenliner und dem Außenliner ausgebildetes Dichtelement zur Anordnung zwischen der Außenseite des Innenliners und der Innenseite des Außenliners ausgebildet ist. Das Dichtelement wird im angelegten Zustand des Linersystems zwischen dem Innenliner und dem Außenliner gehalten. Der Innenliner liegt eng an der Gliedmaße oder dem Gliedmaßenstumpf an, das Dichtelement liegt eng an dem Innenliner an und wird von dem Außenliner abgedeckt. Der Außenliner muss nicht die gesamte Außenseite des Innenliners abdecken. Das zwischen dem Innenliner und dem Außenliner angeordnete Dichtelement dient dazu, die Materialdicke im Bereich des Dichtelementes zu vergrößern, so dass sich an der Außenseite des Außenliners eine vergrößerte Flächenpressung einstellen kann. Die zunächst bevorzugt glattwandige Außenseite des Außenliners wird durch das Dichtelement zwischen dem Innenliner und dem Außenliner konturiert. Aufgrund der separaten Ausgestaltung des Dichtelementes von dem Innenliner und dem Außenliner ist es möglich, ein Linersystem bereitzustellen, insbesondere einen Liner für eine Anwendung einer Prothese an der unteren Extremität, bei dem das Dichtelement variabel einsetzbar und ansetzbar ist. Dadurch ist es möglich, einerseits einen Unterdruck in einem Prothesenschaft sicher herstellen und halten zu können, indem in einem Bereich zwischen einem distalen Ende und einem proximalen Rand eines Prothesenschaftes eine innenliegende Dichtung mit einer erhöhten Flächenpressung bereitgestellt wird, und andererseits die Dichthöhe an der Gliedmaße oder an dem Gliedmaßenstumpf individuell wählbar auszugestalten. Durch die im Wesentlichen freie Wählbarkeit der Position des Dichtelementes auf dem Innenliner ist es möglich, eine einfache Anpassung an die jeweiligen Gegebenheiten an der Gliedmaße oder dem Stumpf oder an das Zusammenspiel zwischen der Gliedmaße, dem Gliedmaßenstumpf und der umgebenden prothetischen oder orthetischen Komponente zu erreichen. Das Linersystem sieht erfindungsgemäß eine doppelwandige Ausgestaltung vor, wodurch eine verbesserte Schutzwirkung für die Gliedmaße oder den Gliedmaßenstumpf erreicht werden kann. Das Dichtelement wird zwischen den Lagen des doppelwandigen Liners angeordnet, insbesondere aufgelegt und an der gewünschten Stelle platziert, wobei durch die individuelle Anordnung des Dichtelementes auf der Außenseite des Innenliners keine Vielzahl von Linermodellen mit angeformten Dichtlippen benötigt werden. Der Außenliner kann kürzbar ausgebildet sein, um eine Anpassung an die jeweilige Körpergröße des Nutzers vornehmen zu können, so dass keine Beeinflussung der Flexionsbewegung durch das Linersystem erfolgt. Bei transtibialen Amputationen kann der Liner unterhalb der Kniekehle gekürzt werden, wodurch eine Beeinflussung der Knieflexion vermieden werden kann.

Das Dichtelement ist ringförmig ausgebildet, wobei eine Vielzahl von unterschiedlichen Innen- und Außendurchmessern des ringförmigen, mit einem geschlossenen Umfang versehenen Dichtelementes vorgesehen sein können, um auf einfache und kostengünstige Art und Weise eine Anpassung an unterschiedliche Einsatzbedingungen erreichen zu können. Durch eine Vielzahl von einfach herzustellenden, preiswerten Dichtelementen in Gestalt von Dichtringen kann bei weitgehend standardisierten Innenlinern und Außenlinern durch einen Anwender oder Orthopädietechniker kostengünstig eine Anpassung an den jeweiligen Patienten erfolgen, indem der jeweils geeignete Dichtring oder das geeignete Dichtelement ausgewählt und eingesetzt wird.

Alternativ zu einem ringförmigen, mit einem geschlossenen Umfang ausgebildeten Dichtelement kann das Dichtelement auch bandartig ausgebildet sein, wobei das Dichtelement dann um den Umfang, bevorzugt vollständig um den Umfang des Innenliners herumgelegt wird, um eine bevorzugt umlaufende Abdichtung und Querschnittsvergrößerung in dem Bereich des Dichtelementes zu erreichen. Nach Auflage und Anordnung des Außenliners über dem Dichtelement wird eine über die Längserstreckung des Linersystems partielle Vergrößerung des Außendurchmessers erreicht, um in diesem Bereich eine verbesserte Dichtwirkung durch eine erhöhte Flächenpressung des Außenliners an den Innenumfang des Schaftes oder der anlegenden orthopädietechnischen Einrichtung zu erreichen. Bei einer bandartigen Ausgestaltung des Dichtelementes besteht die Möglichkeit, dass freie Enden ausgebildet sind, die nicht zu einem geschlossenen Querschnitt zusammengefügt werden müssen, in einer Variante ist es vorgesehen, dass freie Enden eines bandartigen Dichtelementes ineinander überlappend oder nach Art einer Wendel an der Außenseite des Innenliners angeordnet sind, so dass sich insgesamt ein geschlossener Umfang ergibt, wenn in Längserstreckung des Innenliners auf das Dichtelement geschaut wird. Die freien Enden können übereinander gelegt werden und in einem spitzen Winkel einander schneiden, alternativ sind die freien Enden in Axialerstreckung des Innenliners nebeneinander gelegt. Die freien Enden können aneinander angelegt sein oder auch in einem Abstand in Axialrichtung oder Längserstreckung des Innenliners voneinander beabstandet auf den Innenliner positioniert werden.

An dem Dichtelement können Befestigungselemente ausgebildet oder daran befestigt sein, um freie Enden eines bandförmigen Dichtelementes miteinander zu koppeln. Die Kopplung kann formschlüssig erfolgen, beispielsweise durch Ineinanderstecken, Einrasten oder durch Verhaken von an dem Dichtelement ausgebildeten Hinterschneidungen miteinander. Befestigungselemente oder zumindest ein Befestigungselement können auch separat ausgebildet und an dem Dichtelement angeordnet sein, beispielsweise in Gestalt einer Klebeschicht oder eines doppelseitig wirksamen Klebebandes. Das Dichtelement kann an Rastpunkten oder Rastflächen auf der Außenseite des Innenliners befestigt oder positioniert werden. Die Rastpunkte oder Rastflächen können verhindern oder es zumindest erschweren, dass sich das Dichtelement bei einer Benutzung verschiebt.

Der Querschnitt des Dichtelementes kann massiv ausgebildet sein, um eine Kompression und damit eine Verringerung des Querschnittes des Dichtelementes beim Anlegen des Schaftes über den Außenliner zu vermeiden. Hierdurch wird eine hohe Flächenpressung erreicht, allerdings kann dadurch ein erhöhter Druck auf den Innenliner und damit auf die Gliedmaße oder den Gliedmaßenstumpf ausgeübt werden. Der Querschnitt kann auch als Hohlquerschnitt ausgebildet sein, um eine Kompression und Querschnittsverringerung des Dichtelementes zu erreichen. Bei einem geschlossenwandigen Hohlquerschnitt wird eine Kompression durch Verformung ermöglicht. Durch das in dem Dichtelement eingeschlossene Volumen oder die elastische Ausgestaltung des Dichtelementes wird eine Rückstellkraft ausgeübt, so dass eine Druckkraftbegrenzung sowie ein Ausgleich von Volumenschwankungen erreicht werden kann. Der Querschnitt des Dichtelementes kann auch als offenes Hohlprofil ausgebildet sein, beispielsweise L-förmig oder dreieckig mit einem dachartig ausgebildeten, nach außen abstehenden Bereich, wodurch eine erleichterte Verformung des Querschnittes bis zur Auflage des Dachabschnittes an einen Basisabschnitt erreicht werden kann. Ist die Basis erreicht und liegt der Dachabschnitt auf, ist ein erhöhter Verformungswiderstand vorhanden, so dass sich bei einer zunehmenden Kompression von außen ein vergrößerter Widerstand einstellt. Es ist möglich, sämtliche Querschnittsformen, also massiv, als geschlossener Hohlquerschnitt oder als offener Querschnitt oder offener Hohlquerschnitt miteinander zu kombinieren, gegebenenfalls auch nur zwei der Querschnittsformen, um die jeweiligen Eigenschaften des gewählten Querschnittes für das Dichtelement bereichsweise bereitstellen zu können.

Das Dichtelement kann aus einem Schaumstoff gebildet oder mit einem Schaumstoff oder mit einem Textil versehen oder gefüllt sein. Insbesondere über eine Ausgestaltung des Dichtelementes als ein Hohlquerschnitt, sei es ein geschlossener oder ein offener Hohlquerschnitt, kann der Hohlquerschnitt ganz oder teilweise ausgeschäumt oder mit einem Volumenelement, beispielsweise einem Textil oder einem Schaumwerkstoff versehen oder gefüllt werden. Das Textil kann insbesondere als ein textiles Abstandsgewirk ausgebildet sein, das eine Volumenvergrößerung bewirkt, ohne die Kompressibilität oder Verformbarkeit des Dichtelementes wesentlich zu verändern. Je nach Stattlichkeit des Textils oder des Abstandsgewirkes kann die Festigkeit des Dichtelementes variiert werden. Gleiches gilt für die vollständige oder teilweise Füllung des Hohlquerschnittes mit einem Schaumstoff. Statt einer Füllung mit einem Schaumstoff ist es in einer Weiterbildung der Erfindung vorgesehen, dass das Dichtelement selbst aus einem geschäumten Werkstoff, einem Schaumstoff besteht, um ein Volumen zwischen dem Innenliner und dem Außenliner zu erzeugen, um eine Dichtwirkung zu erzielen.

In einer anderen Ausführungsform kann das elastische Volumenelement mit einer Beschichtung versehen sein, die den Reibkoeffizienten des Dichtelements erhöht und somit eine ortsfeste Position des Dichtelements auf dem Innenliner sicherstellt. Bevorzugt weist das elastische Dichtelement eine geringe Härte auf bzw. ist gut komprimierbar, um den Druck auf den Patientenstumpf partiell nicht zu stark zu erhöhen. Der Dichtring kann auch aus einem mit Fluid befüllbaren oder befüllten Hohlkörper bestehen. Durch das Fluid kann das Volumen oder die Eigenschaften des Dichtrings zusätzlich an die Bedürfnisse des Nutzers angepasst werden. Hierzu kann der Hohlkörper mit einer Flüssigkeit oder einem kompressiblen Fluid wie Luft gefüllt werden.

Die Querschnittsform des Dichtelementes kann auch rund, dreieckig, viereckig oder polygonal, gegebenenfalls mit einer flachen Anlagefläche ausgebildet sein, so dass über unterschiedliche Querschnittsformen des Dichtelementes unterschiedliche Anlageeigenschaften und somit auch Dichteigenschaften für das Linersystem eingestellt werden können. Über die Länge oder den Umfang des Dichtelementes kann sich die Querschnittsform ändern, neben kreisförmigen Querschnitten kann der Querschnitt auch abgerundet, oval oder mit einer flachen Anlagefläche und sich einer daran anschließenden Rundung ausgebildet sein.

Bevorzugt besteht das Dichtelement aus einem elastischen Material, insbesondere aus einem Elastomer, beispielsweise Silikon, um insbesondere bei einer Ausgestaltung als ringförmiges Dichtelement durch Aufweiten des Dichtelementes beim Anlegen und Auflegen auf die Außenseite des Innenliners eine sichere Festlegung und Positionierung auf dem Innenliner zu erreichen, bevor das Dichtelement durch den Außenliner nach außen abgedeckt wird. Dadurch, dass das Dichtelement oder der Dichtring aus einem radial leicht dehnbaren Material besteht, kann der Liner für das leichtere An- oder Auszeihen auf- und/oder abgerollt werden. Das Dichtelement oder der Dichtring kann hierzu, neben einem Elastomer, vorzugsweise aus einem Schaumstoff bestehen. Auch andere Volumenelemente, wie beispielsweise eine weichelastische Spiralfeder, die radial aufweitbar ist, sind als Dichtelemente geeignet.

Um ein Verlagern des Dichtelementes nach dem Anlegen an den Innenliner zu vermeiden, ist in einer Weiterbildung der Erfindung vorgesehen, dass das Dichtelement auf der Außenseite des Innenliners und/oder der Innenseite des Außenliners haftet. Dementsprechend ist das Dichtelement aus einem entsprechenden Material ausgebildet oder weist zumindest bereichsweise eine Beschichtung auf, die ein Anhaften an dem Innenliner und/oder Außenliner ermöglicht.

Eine Weiterbildung der Erfindung sieht vor, dass der Innenliner und/oder Außenliner jeweils oder gemeinsam einen geschlossenen, distalen Endbereich aufweisen, so dass sie insbesondere bei einer Ausgestaltung als Prothesenliner über das distale Stumpfende gelegt werden können und dort einen distalen Abschluss bilden. Sofern der Innenliner und Außenliner geschlossenwandig ausgebildet ist, wird durch den jeweils geschlossenen distalen Endbereich ein einseitig offener, mit einer Einführöffnung versehener, hülsenförmiger Liner bereitgestellt, der über den jeweiligen Stumpf gezogen werden kann. In der Regel wird dazu der Innenliner bzw. der Außenliner mit der Innenseite nach außen gedreht, sozusagen auf links gedreht. Die Stumpfspitze wird auf den vorderen Endbereich des umgestülpten Liners aufgesetzt, anschließend wird der übrige Liner auf den Stumpf aufgerollt oder auf ihn aufgezogen, so dass die ursprüngliche Innenseite wieder auf der dem Stumpf zugewandten Seite liegt. Sind sowohl Innenliner als auch Außenliner mit einem geschlossenen, distalen Endbereich versehen, ist auch in diesem besonders von Druckkräfte belasteten Bereich ein doppelwandiger Liner vorhanden, über den Druckkräfte aufgenommen und verteilt werden können. Der distale Endbereich bzw. die distalen Endbereiche können eine zusätzliche Polsterung aufweisen oder eine gegenüber dem übrigen Liner vergrößerte Materialstärke aufweisen, um einen verbesserten Polstereffekt und eine vergrößerte Formstabilität bereitzustellen. Grundsätzlich besteht auch die Möglichkeit, dass nur der Innenliner oder der Außenliner geschlossenwandig ausgebildet ist, um eine Abdichtung des Hohlraumes zwischen dem Prothesenschaft oder der orthopädietechnischen Einrichtung, die um den Liner herum angelegt wird, zu erreichen.

In einer Variante der Erfindung sind der Innenliner und der Außenliner aneinander festgelegt, um das Anlegen zu erleichtern. Zum Anlegen werden dann der Innenliner und der Außenliner gemeinsam von innen nach außen gestülpt, wie oben beschrieben, um dann gemeinsam an dem Stumpf angelegt zu werden.

Eine vorteilhafte Weiterbildung der Erfindung sieht vor, dass der Innenliner und der Außenliner in ihren distalen Endbereichen formschlüssig oder stoffschlüssig miteinander verbunden sind und im distalen Endbereich eine Verbindungszone ausbilden, in der es nicht ohne weiteres möglich ist, ein Dichtelement zwischen den Innenliner und den Außenliner einzulegen. Bei einer stoffschlüssigen Ausgestaltung mit einem elastomeren Werkstoff sind Innenliner und Außenliner miteinander vernetzt, wobei die stoffschlüssige Verbindung miteinander auch durch Vernetzen bereits zweier teilvernetzter Komponenten, also teilvernetzter Innen- und Außenliner hergestellt werden kann. Grundsätzlich ist es auch möglich, dass der Innenliner und der Außenliner in einem gemeinsamen Herstellverfahren gegossen werden. Der Zwischenraum zwischen dem Innenliner und dem Außenliner, in den dann das Dichtelement eingelegt wird, wird durch ein Trennelement ausgebildet, so dass die Verbindungszone ein einteiliger, distaler Endbereich darstellt, an dem sich proximal zwei in der Regel dünnerwandige Innenlinerabschnitte und Außenlinerabschnitte anschließen. Der Innenliner kann mit dem Außenliner bereichsweise verklebt oder verschweißt sein. Ebenso können der Innenliner und der Außenliner reversibel aneinander festgelegt sein, beispielsweise über haftende Eigenschaften des Elastomers oder Formschlusselenente, Klettverschlüsse oder andere Verhakungs- oder Verriegelungselemente, über die der Außenliner lösbar an dem Innenliner festgelegt werden kann. Der Innenliner kann an dem Außenliner auch dauerhaft über Formschlusselemente an dem Innenliner befestigt sein. Im proximalen Bereich des Außenliners sind der Innenliner und der Außenliner nicht miteinander verbunden, um einen ausreichend großen Bereich zu Anordnung eines Dichtelementes bereitzustellen.

Zur Erhöhung des Tragekomforts weisen der Innenliner und/oder der Außenliner einen geschlossenen Querschnitt auf, um Druckstellen bei Überlappungen zu vermeiden. Grundsätzlich ist es auch möglich, einen offenen Querschnitt mit einander überlappenden, mit verringerten Materialstärken versehenen Endbereichen vorzusehen, so dass sich keine Aufdickung in dem Überlappungsbereich einstellt. Ein geschlossenwandiger, hülsenartiger Aufbau sowohl des Außenliners als auch des Innenliners erleichtern sowohl das Anlegen als auch das Tragen und stellen sich, dass eine weitgehende Gasdichtigkeit gegeben und erhalten bleibt. Der Innenliner kann auch einen offenen Querschnitt aufweisen, die einander gegenüberliegenden Kanten stoßen dann bevorzugt nach dem Anlegen aneinander an. Das Dichtelement fixiert dann die Position der Kanten zueinander. Nach dem Überstreifen des Außenliners wird die Zuordnung der Komponenten zueinander weiter gesichert.

Bevorzugt sind der Außenliner oder der Außenliner und der Innenliner aus oder mit einem Elastomer ausgebildet, beispielsweise mit Silikon oder einem anderen Elastomer. Für das sichere Funktionieren ist insbesondere wichtig, dass der Außenliner aus einem Elastomer hergestellt ist oder eine Beschichtung auf seiner Außenseite aufweist, die elastisch und gasdicht ist, um eine sichere Abdichtung zwischen der Außenseite des Außenliners und der Innenseite eines Schaftes oder einer Aufnahmehülse zu gewährleisten. Über eine elastische oder elastomere Ausgestaltung des Außenliners bzw. des Außenliners und des Innenliners ist es möglich, Volumenschwankungen des Stumpfes oder der Gliedmaße auszugleichen oder aber auch unterschiedliche Abmessungen von Gliedmaßen oder Stümpfen mit einem Linermodell oder einer Linergröße versorgen zu können. Dadurch wird es möglich, mit einer überschaubaren Anzahl an Linern eine große Variation an Gliedmaßen oder Stümpfen versorgen zu können.

Bevorzugt bestehen der Innenliner und der Außenliner aus dem gleichen Material, grundsätzlich ist es auch möglich, dass der Innenliner und der Außenliner aus unterschiedlichen Materialen bestehen, beispielsweise um unterschiedliche Anforderungen durch unterschiedliche Materialien erfüllen zu können. So kann für den Innenliner eine erhöhte Atmungsaktivität oder auch Permeabilität für Luft oder Feuchtigkeit vorteilhaft sein, um den Tragekomfort zu erhöhen, während es für den Außenliner wesentlich ist, eine erhöhte Gasdichtigkeit und Formstabilität bereitzustellen, um eine sichere Anlage und Abdichtung gegenüber dem Schaft oder der außen anliegenden Hülse oder Manschette zu gewährleisten. Der Innenliner kann aus einem luftdurchlässigen Material bestehen, das auf seiner Innenseite zumindest bereichsweise mit einer haftenden Beschichtung versehen ist, um eine sichere Zuordnung zu der Hautoberfläche zu gewährleisten. Es ist vorgesehen, dass in einer Variante der Erfindung der Innenliner aus einem Textil besteht, das insbesondere aus einem atmungsaktiven Textil besteht, so dass Feuchtigkeit von der Hautoberfläche weg geleitet werden kann, so dass der Tragekomfort erhöht wird. Für den Außenliner ist die ortsstabile Anlage an dem Innenliner und die Abdichtung gegenüber den außen anliegenden Schaft wesentlich, so dass durch eine Kopplung mit der Außenseite des Innenschaftes über z.B. Verhakungselemente und einer gasdichten Beschichtung bei einer insgesamt elastischen Ausgestaltung des Außenliners durch TPE, Silikon, Polyurethan oder sonstige, geeignete Elastomere eine ausreichende Funktionalität sichergestellt wird. In dem Innenliner und/oder Außenliner kann eine Verstärkungslage eingearbeitet sein, die in eine Trägermatrix, beispielsweise aus einem Elastomer, eingebettet oder auf dieser aufgebracht und mit ihr verbunden ist.

Bei einer Verbindung von Außenliner und Innenliner im Bereich einer Verbindungszone ist es vorteilhaft, wenn sich die Verbindungszone in Proximalrichtung nicht weiter als bis über das erste distale Drittel des Außenliners oder des Innenliners erstreckt, um eine ausreichende Variabilität bei der Positionierung des Dichtelementes zwischen dem Innenliner und dem Außenliner zu erreichen. Dadurch ist es möglich, durch die Herstellung einiger weniger Linerlängen mit einem relativ langen Trennungsbereich zwischen Innenliner und Außenliner im Proximalabschnitt das Dichtelement beliebig oder nahezu beliebig an den gewünschten Stellen und in der gewünschten Orientierung zu positionieren. Dadurch wird die Individualisierbarkeit erhöht und das Linersystem universell einsetzbar, lediglich die Länge muss durch Kürzen angepasst werden.

Auf dem Innenliner können Markierungen zur Ausrichtung des Dichtelementes auf dem Innenliner angeordnet oder ausgebildet sein. Die Markierungen sind insbesondere optische Markierungen, grundsätzlich ist es auch möglich, dass Rillen oder Einkerbungen oder auch Erhebungen auf der Außenseite des Innenliners angeordnet sind, um eine Orientierung für den Orthopädiemechaniker oder auch den jeweiligen Anwender zu erleichtern. Über den Markierungen können auch Orientierungen oder auch Längenabmessungen, insbesondere die Positionierung von einem distalen Ende des Linersystems, einfach kenntlich gemacht werden, um so eine wiederholbare, korrekte Anlegung des Dichtelementes zu gewährleisten.

Die Innenseite des Innenliners bildet zusammen mit dem Außenliner bevorzugt eine glattwandige Innenoberfläche aus, um Druckstellen oder ein Einklemmen oder Faltenbildung mit der Haut der Gliedmaße oder des Gliedmaßenstumpfes zu vermeiden. Bei einer distal geschlossenen Ausgestaltung des Innenliners mit nur einer Einführöffnung am proximalen Ende ist die glattwandige Oberflächengestaltung des Innenliners ohnehin gegeben. Bei einer Zusammensetzung des Systems aus einem Innenliner mit einem Außenliner kann der Außenliner eine geschlossene distale Kappe ausbilden, die in einen Absatz mündet, der im Wesentlichen der Materialstärke eines lediglich hülsenartigen Innenliners entspricht, so dass der Innenliner und der Außenliner beim Anlegen so miteinander positioniert und gefügt werden, dass der Innenliner an den Absatz anstößt und eine glattwandige Innenoberfläche ausbildet, um den Tragekomfort zu erhöhen. Der Innenliner kann dann als Hülse ohne distale Endkappe ausgebildet sein.

Das erfindungsgemäße Verfahren zum Anlegen eines wie oben beschriebenen Linersystems, insbesondere Prothesenlinersystems, sieht zunächst den Schritt des Anlegens eines Innenliners um eine Gliedmaße oder einen Gliedmaßenstumpf vor. Anschließend wird ein ringförmig oder bandartig ausgebildetes, separates Dichtelement auf eine Außenseite des Innenliners aufgelegt oder das Dichtelement auf der Außenseite des Innenliners positioniert. Nach dem Auflegen des Dichtelementes wird ein Außenliner auf der Außenseite des Innenliners angeordnet und das Dichtelement überdeckt. Durch das nachträgliche Anordnen eines separaten Dichtelementes auf der Außenseite des bereits angelegten Innenliners ist es möglich, die Position des Dichtelementes an den jeweiligen Nutzer einfach anzupassen und eine optimale Position individuell einzustellen. Es werden keine Vielzahl von vorgefertigten Prothesenliner und keine individuelle Fertigung eines Prothesenliners mit einer außenliegenden Dichtlippe benötigt, um eine optimale Positionierung des Dichtelementes an dem Patienten vorzunehmen. Die Produktion sowohl des Innenliners als auch des Dichtelementes und des Außenliners ist einfach, es werden keine komplizierten Geometrien für eine Form mit einem angeformten Dichtelement bei der Herstellung eines Liners benötigt. Das Herstellen eines in der Regel hülsenförmigen, glattwandigen, ggf. distal geschlossenen Liners ist einfacher, kostengünstiger und weniger fehleranfällig als die Fertigung eines Liners mit angeformter Dichtlippe. Die Individualisierbarkeit sowohl des Dichtelementes in Form und Positionierung kann durch den Anwender erfolgen, ebenso werden keine neuen Arbeitstechniken für einen Orthopädiemechaniker benötigt. Über unterschiedliche Dichtelemente können Volumenschwankungen einer Gliedmaße leicht ausgeglichen werden. Bei einer Volumenzunahme einer Gliedmaße oder eines Gliedmaßenstumpfes können zur Beibehaltung eines gleichmäßigen Anpressdruckes dünnere oder weichere Dichtelemente eingelegt werden. Bei einer Volumenverringerung können zur Aufrechterhaltung des benötigten Anpressdruckes zwischen der Außenseite des Außenliners und der Innenseite eines Schaftes oder einer Hülse entsprechend steifere oder dickere Dichtelemente eingelegt werden, so dass eine Anpassung ohne große Kosten leicht möglich ist.

Eine Weiterbildung des Verfahrens sieht vor, dass der Außenliner beim Anlegen oder nach dem Anlegen des Innenliners zumindest teilweise die Außenseite des Innenliners überdeckt, also zumindest teilweise über der Außenseite des Innenliners angeordnet ist, beispielsweise um den Außenliner gemeinsam mit dem Innenliner an die Gliedmaße oder den Stumpf anlegen zu können. Anschließend wird ein proximales Ende des Außenliners in distale Richtung heruntergezogen, so dass sich die Innenseite des Außenliners nach außen abrollt, so dass vor dem Anlegen des Dichtelementes die Außenseite des Innenliners bereichsweise freigelegt wird. Anschließend wird das Dichtelement auf die Außenseite des Innenliners aufgelegt und der Außenliner wieder über den Innenliner und das nunmehr darauf angeordnete Dichtelement abgerollt oder darüber gezogen, so dass der Außenliner das Dichtelement überdeckt und damit auf dem Innenliner fixiert. Der Außenliner ist so bemessen, dass er aufgrund seiner elastischen Materialeigenschaften an der Außenseite des Innenliners anliegt, gegebenenfalls leicht komprimierend anliegt, so dass das Dichtelement auf die Außenseite des Innenliners aufgepresst wird. Durch den Außenliner, der sich über den Innenliner und das Dichtelement erstreckt, wird sichergestellt, dass auch bei einem Einführen des Linersystems in einen Schaft oder in eine Hülse das Dichtelement nicht von dem Innenliner durch Scherkräfte verschoben oder abgerollt wird.

In einer Weiterbildung der Erfindung ist vorgesehen, dass das Dichtelement schräg zur Längserstreckung des Innenliners angelegt wird, um eine optimierte Positionierung des Dichtelementes und damit einen optimierten Verlauf der Dichtfläche zwischen der Außenseite des Außenliners und der Innenseite des Schaftes oder der Hülse zu erreichen. Alternativ kann das Dichtelement zu der Längserstreckung des Innenliners auf der Außenseite des Innenliners aufgelegt werden, wodurch sich in der Regel eine Minimierung des Umfangs des Dichtelementes erreichen lässt, so dass die Strecke oder Fläche, auf der das Dichtelement wirken muss, minimiert werden kann.

Eine Weiterbildung der Erfindung sieht vor, dass das Dichtelement nach oder bei dem Auflegen auf die Außenseite des Innenliners an dem Innenliner fixiert wird. Das Dichtelement wird somit beim erstmaligen Anlegen des Innenliners um oder an eine Gliedmaße oder einen Gliedmaßenstumpf auf die Außenseite des Außenliners aufgebracht und dort fixiert, beispielsweise aufgeklebt oder über eine Eigenspannung gehalten, beispielsweise wenn durch Hafteigenschaften eines bandförmigen Dichtelementes, das an Enden oder Bereichen einander überlappend angeordnet wird eine bandförmige Ausgestaltung des Dichtelementes erreicht werden kann. Bevorzugt ist das Dichtelement lösbar an der Außenseite des Innenliners fixiert, um gegebenenfalls die Dichtelemente wieder austauschen zu können, beispielsweise um Abflachungen auszugleichen oder um Dichtelemente an geänderte Bedürfnisse oder einen geänderten Prothesenschaft anpassen zu können. Wenn das Dichtelement dauerhaft an dem Innenliner befestigt wird oder auch lösbar an dem Liner fixiert ist, kann die Positionierung des Dichtelementes auf dem Liner bei einem wiederholten Anlegen des Außenliners entfallen, wodurch dem Patienten oder Nutzer des Linersystems ein Arbeitsschritt gespart wird.

Die Materialstärke des Innenliners und/oder Außenliners ist über den Bereich, bei dem eine zweilagige Ausgestaltung zwischen dem Außenliner und dem Innenliner vorhanden ist, im Wesentlichen gleich oder zumindest kontinuierlich, wobei sich eine im Wesentlichen gleichmäßige Verringerung der Wandstärke von distal nach proximal ergeben kann. Insbesondere sind an der Außenseite des Außenliners keine Vorsprünge oder Dichtelemente angeordnet oder ausgebildet.

Nachfolgend werden Ausführungsbeispiele der Erfindung anhand der beigefügten Figuren näher erläutert. Es zeigen:
- Figur 1: - ein Linersystem mit einem Innenliner und einem Außenliner in einer schematischen Querschnittsansicht;
- Figur 2: - ein Linersystem gemäß Figur 1 mit einem angelegten Dichtelement und einem heruntergeklappten Außenliner;

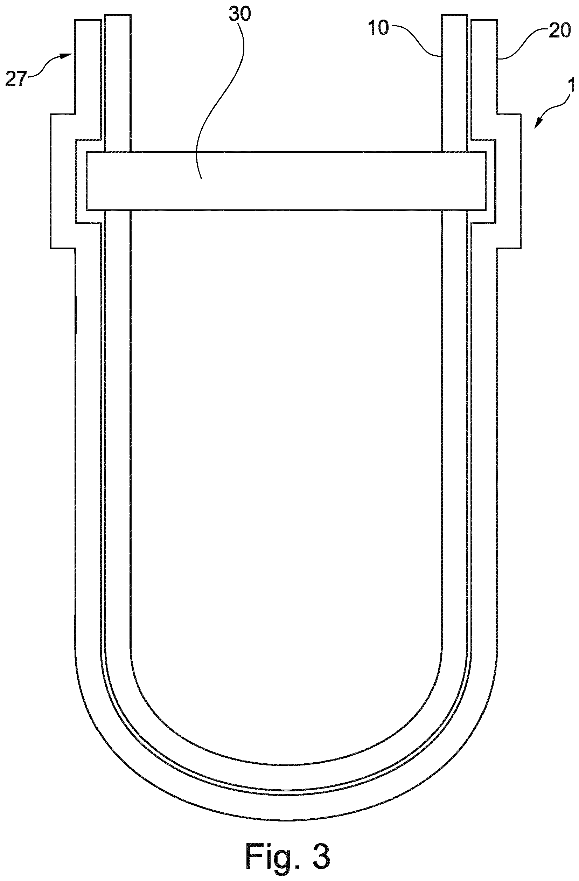

- Figur 3: - ein fertig montiertes Linersystem; sowie
- Figur 4: - eine Querschnittsansicht eines Linersystems ohne Dichtelement mit einer einstückigen Verbindungszone;
- Figur 5: - eine Querschnittsansicht eines Linersystems ohne Dichtelement mit zwei separaten Linern;
- Figur 6: - eine Variante der Figur 2;
- Figur 7: - eine Darstellung der Verschieblichkeit des Dichtelementes auf dem Innenliner;
- Figur 8: - eine Querschnittsansicht eines fertig montierten Linersystems;
- Figur 9: - eine Darstellung eines Dichtelementes in perspektivischer Ansicht und Querschnittsansicht;
- Figur 10: - eine Variante eines Dichtelementes;
- Figur 11: - ein fertig montiertes Linersystem mit Dichtelement gemäß Figur 10;
- Figur 12: - ein separates Dichtelement in perspektivischer Ansicht und Schnittdarstellung;
- Figur 13: - ein fertig montiertes Linersystem mit Dichtelement gemäß Figur 12;
- Figur 14: - eine Variante des Dichtelementes;
- Figur 15: - ein fertig montiertes Linersystem mit einem Dichtelement gemäß Figur 14;
- Figuren 16: und 17 - Einzelansichten eines Dichtelementes in perspektivischer Ansicht und Querschnittsansicht;
- Figur 18: - eine Schnittdarstellung einer Variante mit einem Prothesenschaft; sowie
- Figuren 19: bis 21 - Schnittdarstellungen einer Einführbewegung eines Linersystems in einen Prothesenschaft.

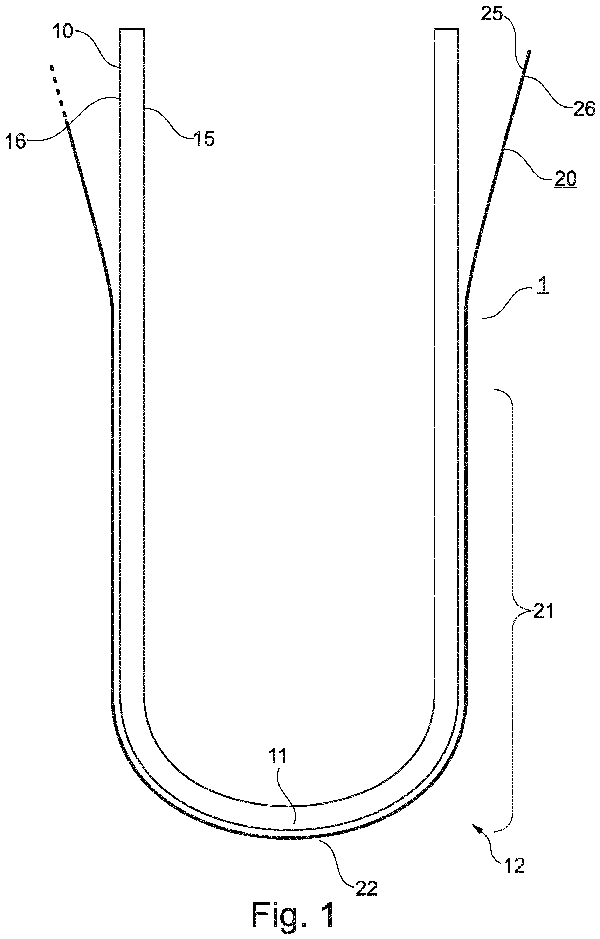

Figur 1 zeigt in einer schematischen Querschnittsansicht einen Teil eines Linersystems 1 mit einem Innenliner 10 und einem Außenliner 20. Der Innenliner 10 weist eine glattwandige Innenseite 15 und eine ebenfalls glattwandige Außenseite 16 auf. Der Innenliner 10 ist hülsenförmig ausgebildet und hat einen distalen, geschlossen ausgebildeten Endbereich 11, so dass sich ein schlauchartiger oder hülsenartiger, geradwandiger oder sich konisch zu einer Einführöffnung hin erweiternder Linerkörper mit einer geschlossenen Endkappe 12 ausbildet. An der Außenseite 16 des Innenliners 10 ist ein Außenliner 20 angeordnet, dessen Innenseite 25 der Außenseite 16 des Innenliners gegenüberliegt. Die Außenseite 26 des Außenliners 20 ist glatt ausgebildet. Die Form und Kontur der Innenseite 25 des Außenliners 20 entspricht im Wesentlichen der Form und Kontur der Außenseite 16 des Innenliners, gegebenenfalls kann die Innenkontur des Außenliners 20 einen geringeren Durchmesser oder Umfang als der Außenumfang des Innenliners 10 aufweisen, um bei einem übereinander angelegten Zustand eine ausreichende Kompression des Außenliners 20 gegen den Innenliner 10 zu gewährleisten. Zur verbesserten Sichtbarkeit ist der Außenliner 20 im proximalen Endbereich, also im Bereich der Einstiegöffnung, sich konisch erweiternd ausgebildet dargestellt. Der Außenliner 20 kann einen rohrförmigen, mit einem konstanten Durchmesser versehenen Querschnitt oder eine sich leicht vergrößernde konische Ausgestaltung aufweisen. Durch die konische Ausgestaltung des Innenliners 10 und des Außenliners 20 ergibt sich eine verbesserte Anlage an einen in der Regel ebenfalls sich konisch in Proximalrichtung vergrößernden Stumpf oder einer Gliedmaße, beispielsweise einen Unterschenkelstumpf, einen Oberschenkelstumpf oder einen Oberarmstumpf.

In dem dargestellten Ausführungsbeispiel ist der Innenliner 10 mit einer größeren Wandstärke als der Außenliner 20 ausgebildet, grundsätzlich ist es auch möglich, dass sowohl der Innenliner 10 als auch der Außenliner 20 eine gleiche Materialstärke aufweisen oder der Außenliner 20 dicker als der Innenliner 10 ausgebildet ist.

Der Außenliner 20 ist in einem distalen Endbereich 21 an dem Innenliner 10 festgelegt, so dass sich eine Verbindungszone 21 ausbildet, in der der Außenliner 20 mit einer Innenseite an der Außenseite 16 des Innenliners 10 fest verbunden ist. Die Verbindung kann durch Kleben, Verschweißen, punktuelles Verbinden oder auch durch Vernetzen oder ein einstückiges Ausgestalten durch einen Gießvorgang beim Urformen hergestellt werden. Neben einer einstückigen Ausgestaltung ist es möglich, zunächst den Innenliner 10 und den Außenliner 20 separat voneinander herzustellen und dann miteinander formschlüssig oder stoffschlüssig miteinander zu verbinden. In dem dargestellten Ausführungsbeispiel sind der Innenliner 10 und der Außenliner 20 beide mit einem geschlossenen distalen Endbereich 11, 22 versehen. Es besteht auch die Möglichkeit, dass nur einer der beiden Liner 10, 20 mit einer distalen Endkappe 12 versehen ist.

Beide Liner 10, 20 weisen zumindest eine elastomere Materialkomponente auf, wobei zumindest der Außenliner 20 auf seiner Außenseite 26 eine im Wesentlichen luftundurchlässige Beschichtung aufweist oder der gesamte Außenliner 20 aus einem luftundurchlässigen Material besteht.

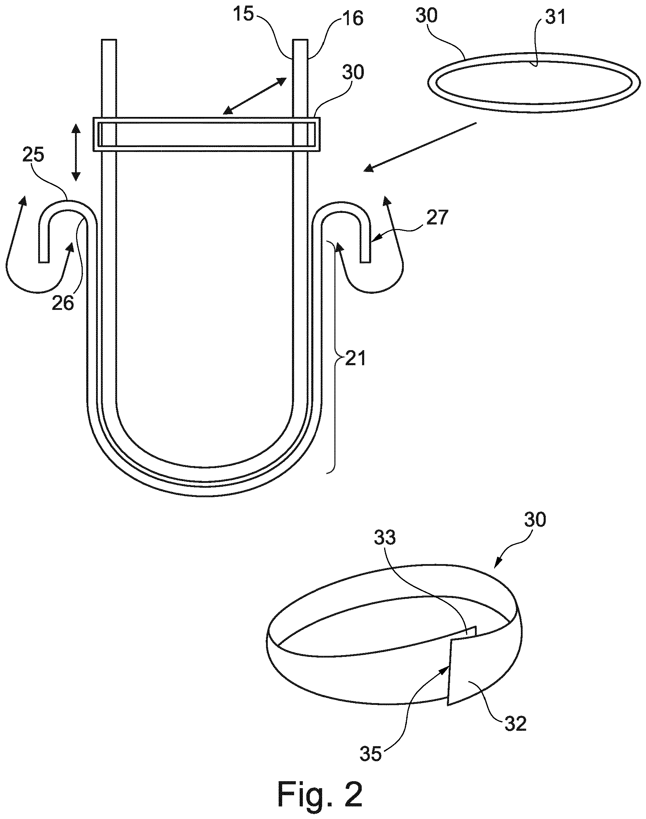

Figur 2 zeigt das Prothesenlinersystem 1 während des Anlegens. Der Innenliner 10 ist bereits an dem nicht dargestellten Stumpf einer Gliedmaße angelegt. Der Außenliner 20 ist von seinem proximalen Ende, also von der Einstiegsöffnung her nach unten in Richtung zum distalen Endbereich 22 bis zum proximalen Ende der Verbindungszone 21 heruntergezogen oder heruntergerollt, so dass die eigentliche Innenseite 25 des Außenliners 20 nach außen zeigt. Ein separates Dichtelement 30, das im dargestellten Ausführungsbeispiel ringförmig ausgebildet ist, ist im Bereich der gewünschten Höhe auf der Außenseite 16 des Innenliners 10 positioniert. Das Dichtelement 30 ist in einer Einzeldarstellung rechts neben den beiden Linern 10, 20 gezeigt. Im dargestellten Ausführungsbeispiel ist der Querschnitt des Dichtelementes 30 rechteckig, grundsätzlich sind auch andere Querschnittsformen des Dichtelementes 30 möglich. Grundsätzlich ist es auch möglich, dass das Dichtelement 30 bandförmig ausgebildet ist und zur Anlage um den Innenliner 10 herum herumgelegt wird, eine solche Ausgestaltung des Dichtelementes 30 ist unterhalb des Prothesenlinersystems 1 gezeigt. Das Dichtelement 30 weist freies Enden 32, 33 auf, die freien Enden 32, 33 können einander überlappen und sind zur Ausbildung einer umlaufenden Verdickung im angelegten Zustand um den Innenliner 10 einander überlappend angeordnet. Dabei können zur Vermeidung einer ungewollten Materialaufdickung sich die freien Enden 3, 33 zum Ende hin verjüngen, so dass bei einer Überlappung der jeweiligen Endbereich des Dichtelementes 30 eine im Wesentlichen gleiche Materialstärke zur Sicherstellung eines gleichmäßigen Außenumfanges des angelegten Dichtelementes 30 sichergestellt wird. Die freien Endbereiche 32, 33 eines bandförmigen Dichtelementes 30 können über Befestigungselemente 35 oder Befestigungseinrichtungen aneinander gesichert werden, beispielsweise über ein Verhaken oder ein Verkleben der Endbereiche.

Das Dichtelement 30 ist reversibel an der Außenseite 16 des Innenschaftes festgelegt und weist eine ebene Anlagefläche 31 auf, um eine flächige und sichere Anlage des Dichtelementes 30 an der Außenseite 16 des Innenliners 10 zu gewährleisten. Bevorzugt ist das Dichtelement 30 ebenfalls elastisch ausgebildet, um Volumenschwankungen des Stumpfes und damit Volumenänderungen und Außenumfangsänderungen des Innenschaftes 10 folgen zu können.

Nach dem Anlegen des Dichtelementes in der gewünschten Position an dem Innenliner 10, wobei die Position über Markierungen an dem Innenliner 10 festgehalten werden kann, um eine reproduzierbare Positionierung des Dichtelementes auch für einen Endanwender leicht zu ermöglichen, wird der Außenliner 20 wieder über den Innenliner 10 abgerollt oder hochgezogen, so dass die Innenseite 25 des Außenliners 20 im Wesentlichen vollflächig über der Außenseite 16 des Innenliners 10 und das Dichtelement 30 anliegt. Im Bereich des Dichtelementes 30 wölbt sich der Außenliner 20 nach außen und stellt dort eine Umfangsvergrößerung bereit, so dass in diesem Bereich eine erhöhte Flächenpressung beim Anlegen eines nicht dargestellten Prothesenschaftes oder einer anderen Einrichtung ausgebildet wird, um dort eine sichere Festlegung über einen Unterdruck im Zwischenraum zwischen dem Außenliner 20 und dem nicht dargestellten Prothesenschaft oder Schaft sicherstellen zu können. In dem angelegten Zustand des Linersystems 1 liegt das separate, reversibel festlegbare und wieder entfernbare Dichtelement 30 zwischen der Außenseite 16 des Innenliners 10 und der Innenseite 25 des Außenliners 20. Mit dem Linersystem 1 ist es möglich, durch nur einen Linertyp eine Vielzahl von Patienten versorgen zu können und eine einfache Anpassung auch an unterschiedliche Schaftsysteme oder Hülsensysteme vornehmen zu können. Der Ort der Dichtung zwischen der Außenseite des Liners und der Innenseite des Schaftes ist durch die variable Positionierung des Dichtelementes 30 zwischen dem Innenliner 10 und dem Außenliner 20 frei wählbar und kann unter Berücksichtigung der anatomischen Gegebenheiten und der konstruktiven Ausgestaltung des Schaftes individuell optimiert werden. Die einmal gefundene, optimierte Position und der Verlauf der Dichtlinie, die durch die auch gegenüber der Längserstreckung geneigte Positionierung des Dichtelementes 30 gegenüber dem Innenliner 10 nahezu beliebig gewählt werden kann, kann durch die reversible Anordnung des Dichtelementes 30 verändert werden. Volumenänderungen über die Tragzeit des Schaftes können durch eine Anpassung des Dichtelementes 30 ausgeglichen werden. Dazu kann eine Vielzahl einfach herzustellender, preiswerter Dichtelemente in unterschiedlichen Dimensionierungen, in unterschiedlichen Querschnitten und in unterschiedlichen Materialien vorrätig gehalten werden, die eine erleichterte Individualisierung ermöglichen.

Bei einer permanenten Befestigung des Außenliners 20 an den Innenliner 10 wird das Anlegen des Linersystems 1 erleichtert. Grundsätzlich ist es auch möglich, dass Außenliner 10 und Innenliner 20 separat ausgebildet sind. Es besteht die Möglichkeit, dass Innenliner 10 und Außenliner 20 identisch ausgebildet sind, wodurch sich die Herstellkosten des Linersystems 1 weiter verringern lassen.

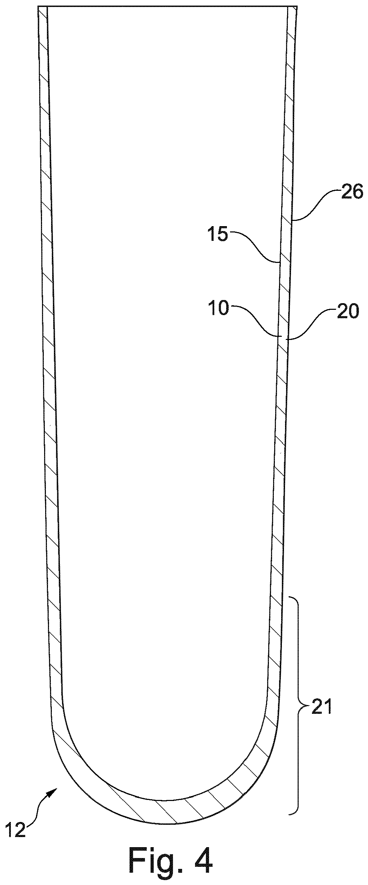

Figur 4 zeigt in einer Querschnittsansicht einen Teil eines Linersystems 1 mit einem Innenliner 10 und einem Außenliner 20, die in einer Verbindungszone 21 im distalen Endbereich mit einer geschlossenen Endkappe 12 ausgebildet sind, wobei innerhalb der Verbindungszone 21 der Innenliner 10 mit dem Außenliner 20 stoffschlüssig durch eine einstückige Ausgestaltung miteinander verbunden sind. Die Verbindungszone 21 erstreckt sich auf zwischen 25% und 30% der Gesamtlänge der Liner 10, 20 in der Ausgangskonfiguration. Das proximale Ende des Linersystems1 kann nach Bedarf gekürzt werden. Der Liner 10 und der Außenliner 20 sind somit nur teilweise über die Länge der Liner 10, 20 voneinander getrennt, beispielsweise durch eine entsprechende Vorrichtung bei der Herstellung.

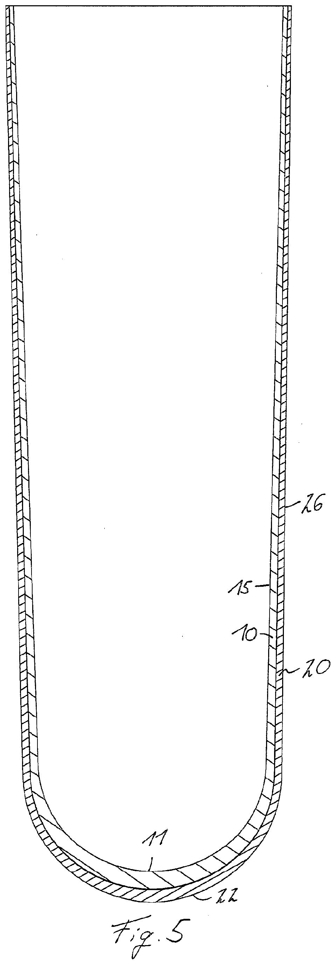

Eine Variante der Erfindung ist in der Figur 5 in Querschnittsdarstellung gezeigt, bei der der Innenliner 10 und der Außenliner 20 als separate Liner hergestellt und aneinander angelegt sind. Gegebenenfalls kann in den distalen Endbereichen 11, 20 eine Verbindung zwischen dem Außenliner 20 und dem Innenliner 10 vorhanden sein, beispielsweise durch bereichsweises Verkleben oder über Befestigungselemente, beispielsweise Formschlusselemente wie z.B. Haken- und Flauschbereiche.

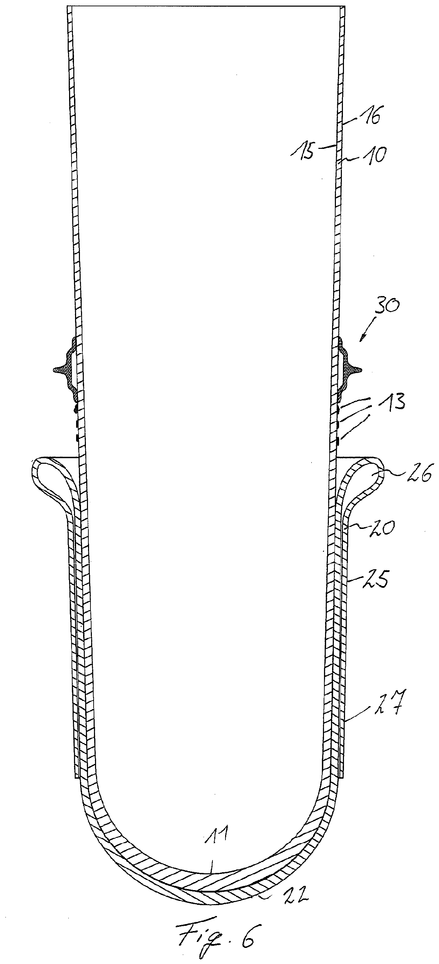

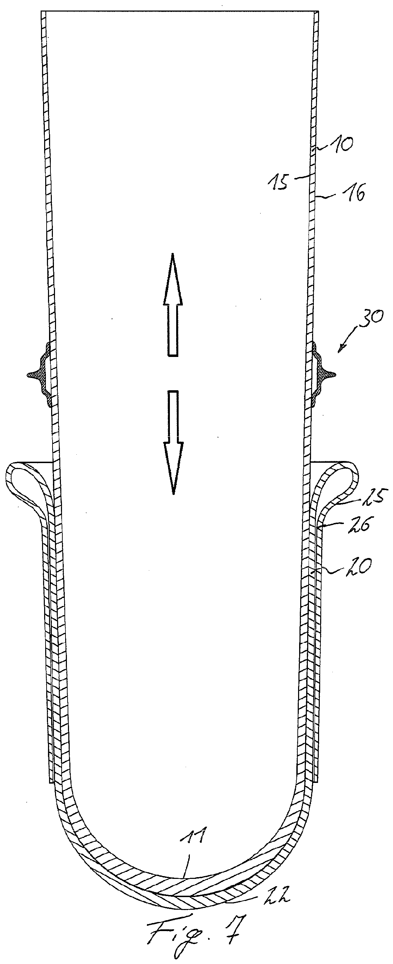

Figur 6 zeigt in einer Querschnittsansicht eine Variante der Figur 2, bei der ein Innenliner 10 und ein Außenliner 20, ähnlich der Darstellung gemäß Figur 5, ineinander gefügt sind, so dass die Außenseite 16 des Innenliners an der Innenseite 25 des Außenliners 20 anliegt. Das proximale Ende 27 oder ein proximaler Endbereich 27 des Außenliners 20 ist von dem proximalen Ende in Richtung auf den distalen Endbereich 22 heruntergezogen, so dass die Außenseite 16 des Innenliners 10 freiliegt. Die Innenseite 25 des Außenliners 20 ist bereichsweise nach außen umgestülpt. An der Außenseite 16 des Innenliners 10 ist ein ringförmiges Dichtelement 30 angeordnet, das zwei Auflageflächen aufweist, die in Längserstreckung des Innenliners 10 zueinander beabstandet sind. Eine radial nach außen gerichtete Zunge ist an dem Dichtelement 30 umlaufend ausgebildet. Das Dichtelement 30 kann haftend, über eine aufgrund einer elastischen Ausgestaltung des ringförmigen Dichtelementes 30 aufgebrachten elastischen Haltekraft oder über eine Klebeverbindung an der Außenseite 16 des Innenliners 10 befestigt und festgelegt sein. Sofern keine klebende Festlegung des Dichtelementes 30 an der Außenseite 16 des Innenliners 10 vorhanden ist, ist das Dichtelement 30 entlang der Längserstreckung des Innenliners 10 an dessen Außenseite 16 verschieblich, wie durch den Doppelpfeil in der Figur 7 angedeutet ist. Die Position des Dichtelementes 30 an dem Innenliner 10 ist somit frei wählbar, eine Anpassung kann an die Bedürfnisse des jeweiligen Patienten in Abstimmung mit einem Orthopädiemechaniker erfolgen. Eine einmal gefundene, als angenehm oder optimal erachtete Position des Dichtelementes 30 auf dem Innenliner 10 kann über eine Markierung dokumentiert werden. Auf der Außenseite 16 des Innenliners 10 können Markierungen 13 ausgebildet, aufgebracht oder eingearbeitet sein, um eine reversible Festlegung des separaten Dichtelementes 30 auf dem Innenliner 10 leicht vornehmen zu können. Die Markierungen sind in der Figur 6 umlaufend dargestellt, sie können auch nur entlang einer oder mehrerer, sich in Längserstreckung des Innenliners 10 verlaufender Linien angeordnet sein. Die Markierungen 13 können auf der Außenseite 16, der Innenseite 15 oder innerhalb des Innenliners 10 angeordnet sein. Es ist auch vorgesehen, dass umlaufende Markierungen 13 nicht senkrecht zu der Längserstreckung des Innenliners 10 verlaufen.

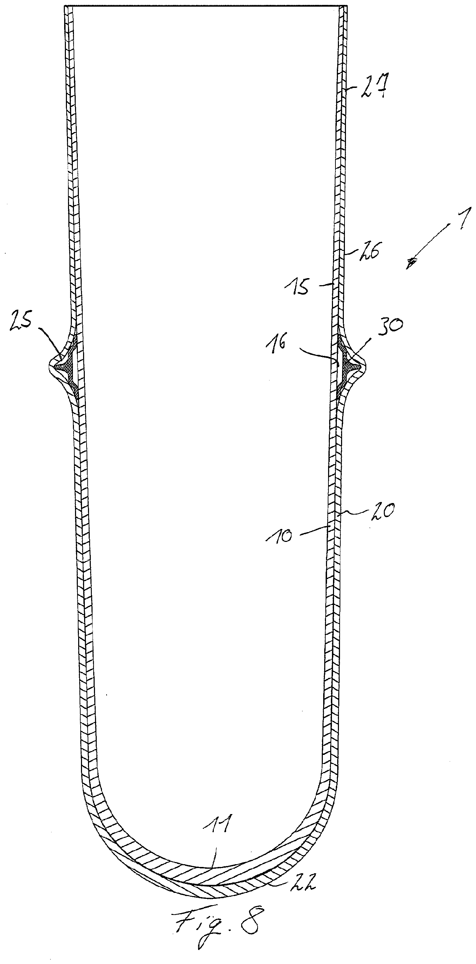

Figur 8 zeigt ein vollständig montiertes Linersystem in einem nicht an einen Stumpf angelegten Zustand. Das proximale Ende 27 des Außenliners 20 ist wieder nach oben umgeschlagen, so dass die Innenseite 25 des Außenliners 20 an der Außenseite 16 des Innenliners 10 anliegt. Der Außenliner 20 überdeckt das Dichtelement 30, wobei sich durch die radial nach außen gerichtete Zunge des Dichtelementes 30 eine Auswölbung im Bereich des Dichtelementes 30 innerhalb des Außenliners 20 einstellt. Sofern ein solches Linersystem 1 mit einem Innenliner 10, einem Außenliner 20 und einem dazwischen angeordneten, separat hergestellten Dichtelement 30 in einen nicht dargestellten formstabilen Prothesenschaft eingeführt wird, wird im Bereich des Dichtelementes 30 eine erhöhte Anpressung des Außenliners 20 an die Innenwand des Prothesenschaftes bewirkt, so dass die Außenseite 26 des Außenliners 20 dichtend an dem Innenumfang des Prothesenschaftes anliegt. Bevorzugt sind die Liner 10, 20 aus einem elastomeren, insbesondere luftundurchlässigen Material hergestellt.

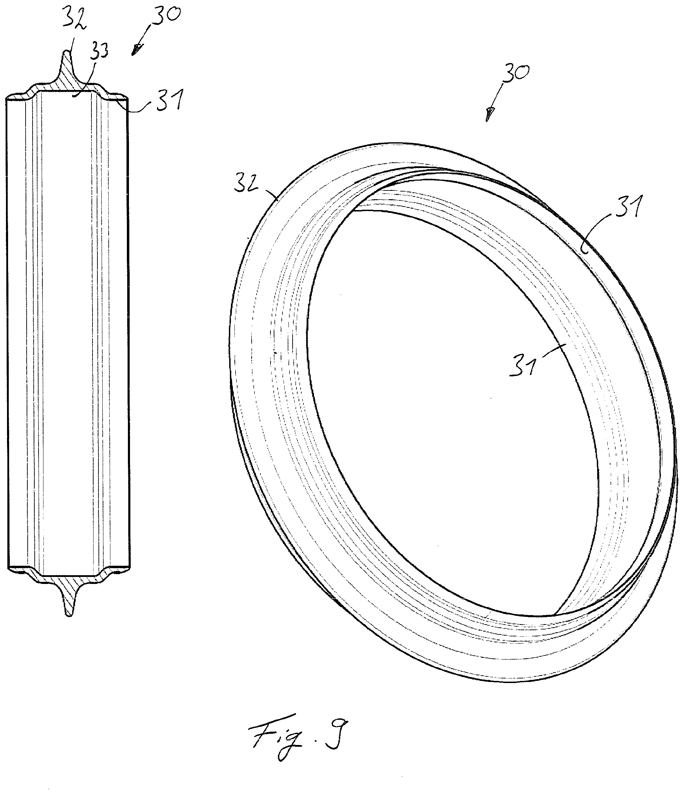

Figur 9 zeigt das Dichtelement 30 gemäß Figur 8 in einer Einzeldarstellung in einer perspektivischen Ansicht sowie in einer Querschnittsansicht. Das Dichtelement 30 ist ringförmig ausgebildet und weist neben zwei im Wesentlichen zylindrischen, glatten Anlageflächen 31 eine nach außen gerichtete, umlaufende Zunge 32 auf. Zwischen den beiden Anlageflächen 31, die zueinander beabstandet sind, ist ein Absatz und ein Freiraum 33 ausgearbeitet, um eine elastische Einfederung der Zunge 32 in Richtung auf die flachen Anlageflächen 31 zu ermöglichen. Der Freiraum 33 ermöglicht ein auch nur bereichsweises Einfedern der Zunge 32 radial nach innen, insbesondere wenn das Dichtelement 30 aus einem elastomeren Werkstoff hergestellt ist. Die Anlageflächen 31 können haftend ausgebildet sein oder mit einer haftenden Beschichtung versehen sein. Die radial nach außen stehende Zunge 32 kann eine gegenüber dem übrigen Material erhöhte Formstabilität aufweisen. Die Zunge 32 kann flexibel, insbesondere elastisch ausgebildet sein, so dass sie sich nach dem Anlegen des Außenliners 20 oder nach dem Einführen des Linersystems in einen Schaft verformt, insbesondere an den Innenliner 10 anlegt.

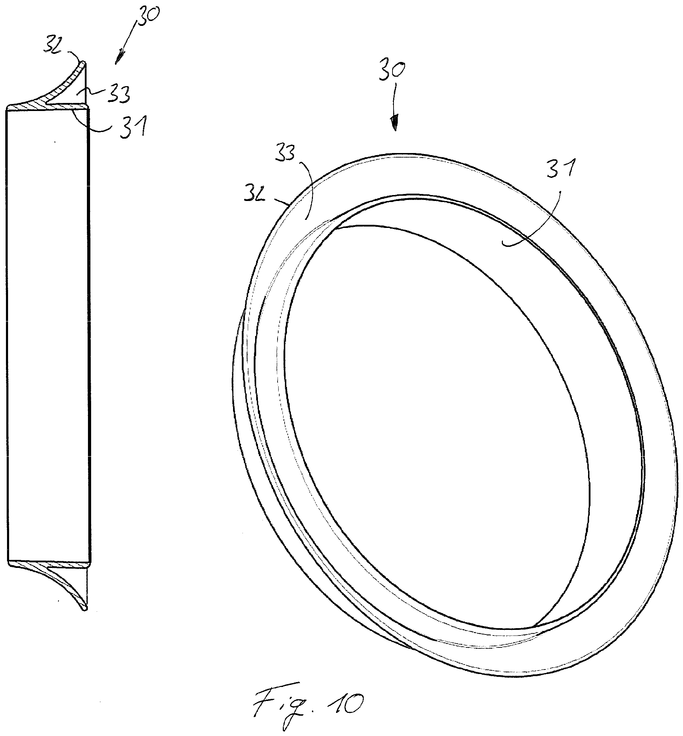

Eine Variante des Dichtelementes 30 ist in der Figur 10 in einer perspektivischen Darstellung und in einer Querschnittsdarstellung gezeigt. Die Anlagefläche 31 ist vollflächig ausgebildet, so dass sich ein Ring oder einem Grundkörper ausbildet, von dem sich in einem Bogen radial nach außen eine umlaufende Zunge 32 nach außen erstreckt. Mit einem äußeren Rand der umlaufenden Zunge 32 bis zu dem umlaufenden Ring, an dessen Innenseite die Anlagefläche 31 ausgebildet ist, ist ein Freiraum 33 ausgebildet, durch den ein Einfedern des äußeren Randes der umlaufenden Zunge 32 radial nach innen ermöglicht wird.

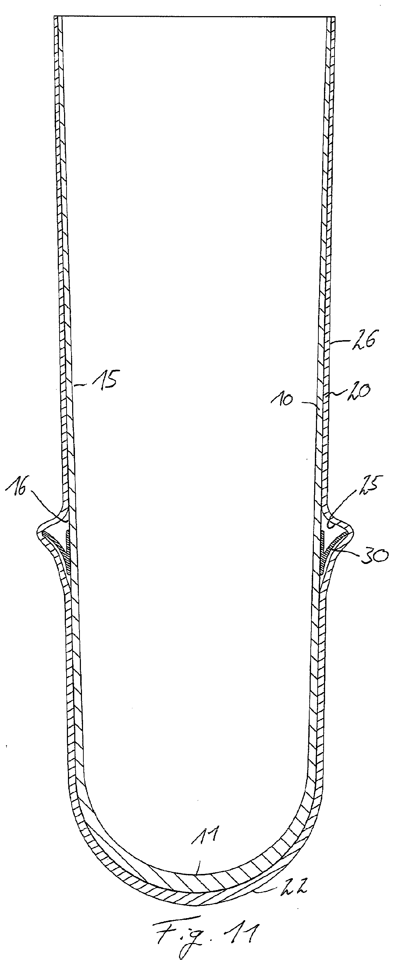

Das montierte Linersystem 1 mit einem Dichtelement 30 gemäß Figur 10 ist in der Figur 11 dargestellt. Das Dichtelement 30 ist so montiert, dass sich eine V-förmige, zum proximalen Ende hin aufweitende Ausgestaltung ergibt, so dass bei einem Einführen des Linersystems 1 in einen Prothesenschaft die nach außen stehende Lippe oder Zunge 32 radial nach innen gedrückt werden kann, wobei sich die Lippe oder Zunge 32 in den Freiraum 33 hineinbewegen lässt und eine elastische Rückstellkraft ausübt, die nach außen wirkt. Die Lippe oder Zunge 32 und der Grundkörper mit der Anlagefläche 31 sind entweder miteinander verbunden oder einstückig miteinander ausgebildet.

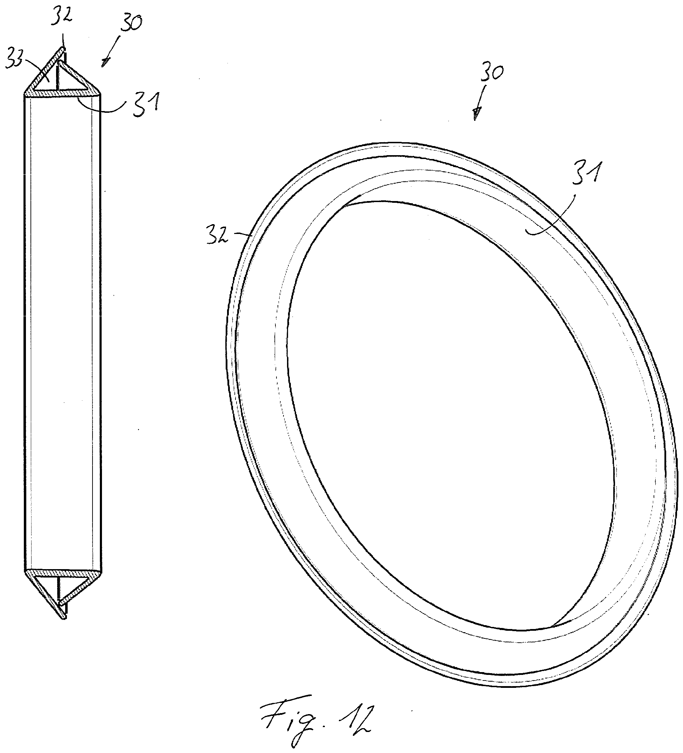

Figur 12 zeigt eine Variante des Dichtelementes 30 mit einem im Wesentlichen dreieckigen Querschnitt, wie in der linken Darstellung der Figur 12 gezeigt ist. Eine bandartige Grundfläche mit einer auf der Innenseite ausgebildeten, flachen Anlagefläche 31 dient zur Anlage an der Außenseite 16 des Innenliners 10. Von der Grundseite erstrecken sich zwei Schenkel aufeinander zu, so dass sich ein im Querschnitt im Wesentlichen dreieckiger Hohlraum 33 ausbildet. In der dargestellten Ausführungsform ist der linke Schenkel länger als der rechte Schenkel, so dass sich ein Überstand ergibt, der eine äußere Kante oder Zunge 32 ausbildet. Aufgrund des Hohlraumes 33 und des Umstandes, dass die beiden von der Grundseite aufeinander zu abragende Schenkel nicht miteinander verbunden sind, kann eine radial nach innen gerichtete Verlagerung bei einer Kompression und einem Druck von außen in Richtung auf die Grundseite oder Anlagefläche 31 erfolgen.

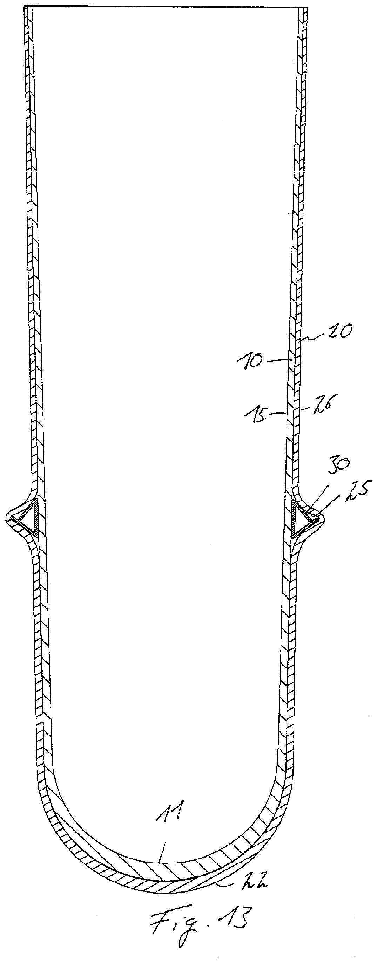

Das fertig montierte Linersystem 1 ist in der Figur 13 gezeigt, bei der das Dichtelement 30 gemäß Figur 12 zwischen dem Innenliner 10 und dem Außenliner 20 angeordnet ist.

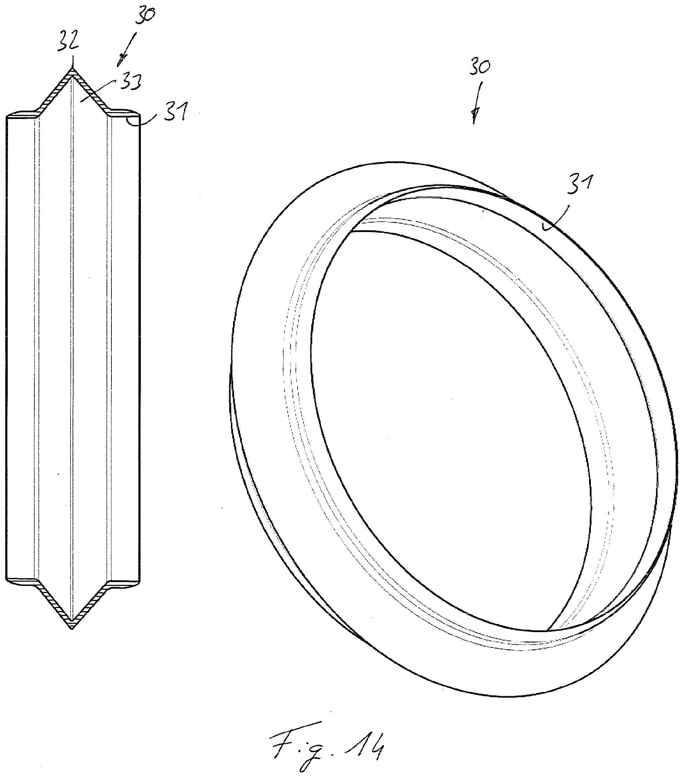

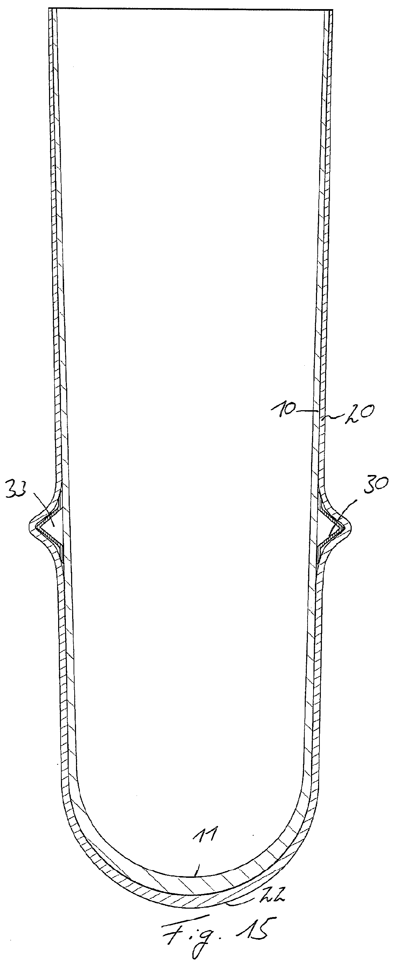

Eine weitere Variante der Erfindung ist in der Figur 14 gezeigt, bei der das Dichtelement ähnlich der Figur 9 zwei Anlageflächen 31 aufweist, von denen sich aus in einem Winkel zwei konische Schenkel radial nach außen erstrecken, so dass eine umlaufende Kante oder radial abstehende Zunge 32 ausgebildet wird. Aufgrund der konischen Ausgestaltung ergibt sich ein Hohlraum 33 zwischen den Anlageflächen 31 und eine im Wesentlichen spitze, dreieckige Ausgestaltung des Dichtelementes 30 im angelegten Zustand. Das fertig montierte Linersystem 1 ist in der Figur 15 zu erkennen, bei dem der Hohlraum 33 im angelegten Zustand im Wesentlich dreieckig ist. Durch die außen umlaufende Kante oder Zunge 32 der konischen Flanken ergibt sich eine Auswölbung des Außenliners 20 im Bereich des Dichtelementes 30.

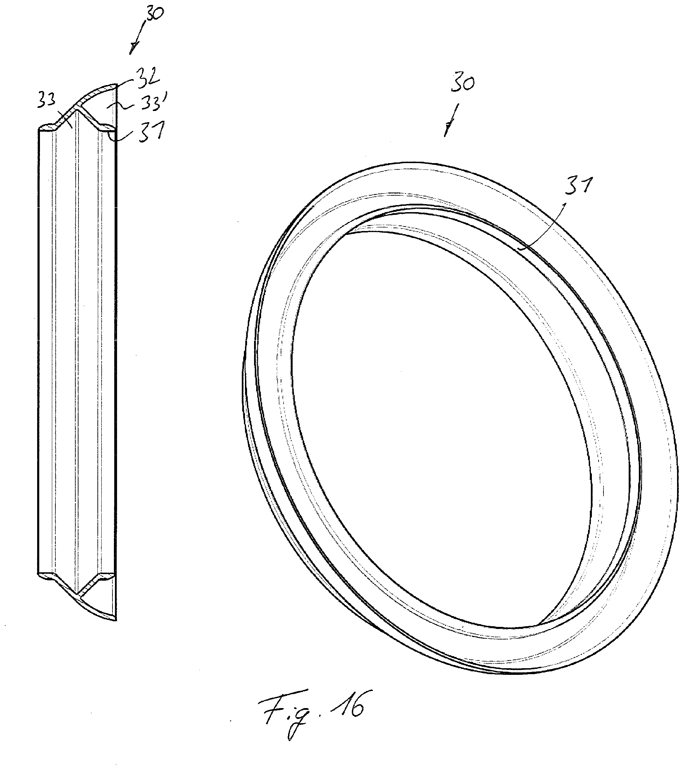

Eine weitere Variante des Dichtelementes 30 ist in der Figur 16 gezeigt, bei der ähnlich der Form gemäß Figur 14 ein erster dreieckiger Hohlraum 33 zwischen den Anlageflächen 31 ausgebildet wird. Im Bereich der dachartigen Verbindungsstelle der beiden konischen Flanken erstreckt sich eine weitere Zunge 32 zu einer Seite des Dichtelementes 30, so dass sich zwischen einer ersten Anlagefläche 31 und dem äußeren Rand 32 ein zweiter Hohlraum 33' ergibt. Eine solche Ausgestaltung kann eine mehrfache Einfederung des Dichtelementes 30 in radialer Richtung erfolgen.

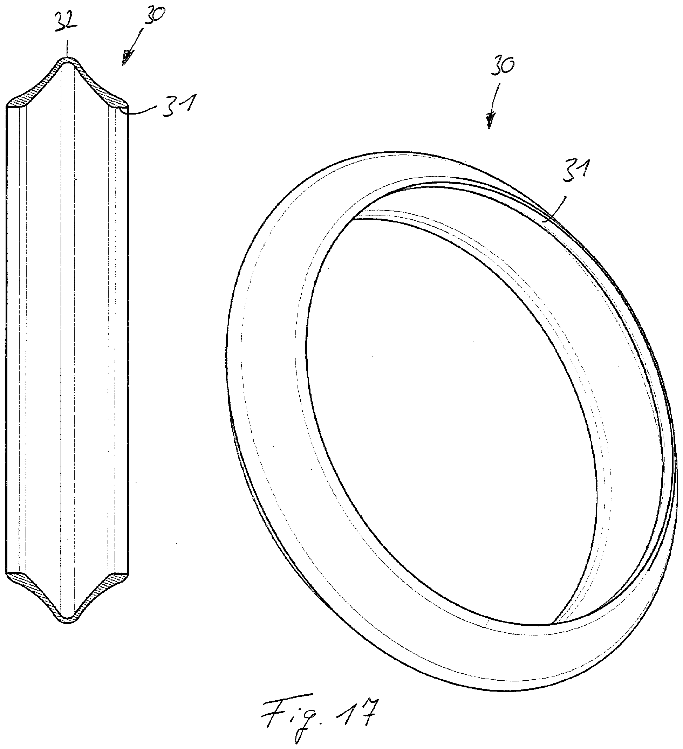

Figur 17 zeigte eine weitere Variante des Dichtelementes mit einer geschwungenen Form. Von den beiden Anlageflächen 31 erstreckt sich bogenförmig und dachartig jeweils ein Schenkel in Richtung auf den äußeren Umfang zur Ausbildung eines Vorsprunges oder einer Zunge 32, die ebenfalls abgerundet ausgebildet ist. Durch die abgerundete Ausgestaltung sowohl der Schenkel als auch der äußeren Umfangskante wird eine abgerundete Auswölbung des Außenliners 20 im fertig montierten Zustand erreicht.

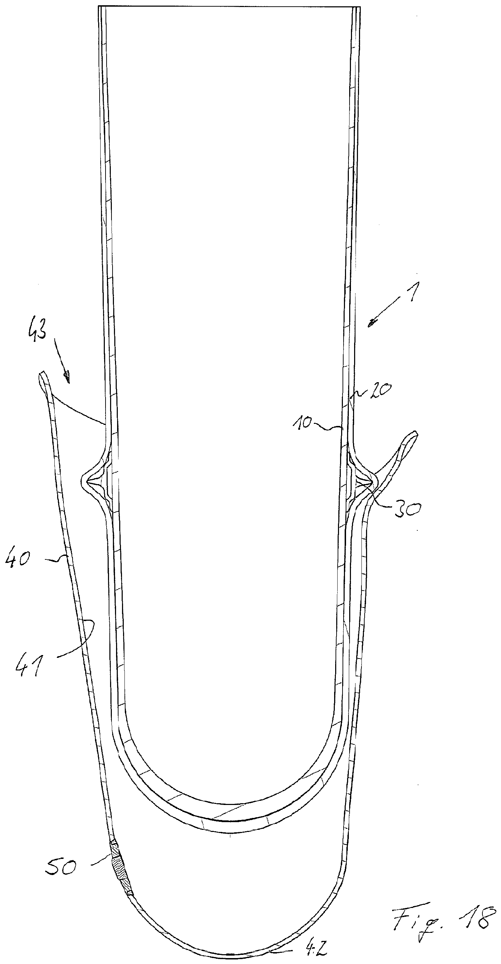

In der Figur 18 ist in einer schematischen Schnittdarstellung ein Prothesenlinersystem 1 mit einem Innenliner 10 und einem Außenliner 20 sowie einen dazwischen angeordneten Dichtelement 30 gezeigt. Der Aufbau entspricht im Wesentlichen dem der Figur 8, wobei das Dichtelement 30 weiter distal als in der Figur 8 angeordnet ist. Das Prothesenlinersystem 1 ist teilweise in einen Prothesenschaft 40 eingeführt, der einen geschlossenen distalen Endabschnitt 42 und eine proximale Einführöffnung 43 aufweist. Der Prothesenschaft 40 weitet sich von dem distalen Endabschnitt 42 zur proximalen Einführöffnung 43 auf, so dass er im Wesentlichen sich konisch erweiternd ausgebildet ist. In dem dargestellten Ausführungsbeispiel weist der Prothesenschaft 40 eine geschlossene Innenwand 41 auf und ist aus einem formstabilen Material hergestellt, beispielsweise einem faserverstärkten Kunststoff, einem Leichtmetall oder einem ähnlich stabilen und leichten Material. An der Außenseite des Prothesenschaftes 40 können Befestigungseinrichtungen für weitere Prothesenkomponenten angeordnet sein, um diese an dem Prothesenschaft 40 festzulegen, beispielsweise Aufnahmen für Anschlussadapter, Anschlussadapter als solche, einlaminierte Anschlussadapter oder einlaminierte Befestigungseinrichtungen wie Schraublöcher, Bolzen oder Befestigungsplatten, um daran beispielsweise Rahmen, Schienen oder direkt Prothesengelenke, insbesondere Prothesenkniegelenke oder Prothesenellenbogengelenke, Prothesenknöchelgelenke oder Prothesenhandgelenke zu befestigen. Grundsätzlich können auch andere Prothesenkomponenten wie Prothesenfüße, Unterschenkelrohre, Unterarmrohre oder dergleichen direkt an dem Prothesenschaft 40 befestigt werden oder befestigt sein.

In der Figur 18 ist das Prothesenlinersystem 1 nicht vollständig in den Prothesenschaft 40 eingeführt, sondern stößt zunächst mit dem nicht komprimierten, vergrößerten Außenumfang des Außenliners 20 an die Innenwand 40 des Prothesenschaftes im Bereich der proximalen Einführöffnung 43 an, ohne dass es zu einer vollumfänglichen Anlage des nach außen hervorstehenden, ausgewölbten Außenliners 20 kommt. Aufgrund der sich konisch in Richtung auf den distalen Abschlussbereich 42 verjüngenden Prothesenschaft 40, wird bei einem weiteren Einführen des Linersystems 1 der Außenliner 20 im Bereich der größten Auswölbung durch den Vorsprung der Dichtlippe 30 umfänglich in Kontakt mit der Innenseite 41 des Prothesenschaftes treten und dadurch ein abgeschlossenes Volumen ausbilden, wenn im Bereich der Dichtlippe 30 der Außenliner 20 vollumfänglich dichtend an der Prothesenschaftinnenwand 41 anliegt. Bei einer weiteren Einführbewegung des Linersystems 1 in Distalrichtung auf den distalen Abschlussbereich 42 des Prothesenschaftes 40 hin, wird die in dem Volumen eingeschlossene Luft zunächst komprimiert. Das in dem dargestellten Ausführungsbeispiel mit einer senkrecht nach außen gerichteten Zunge 32 ausgebildete Dichtelement 30 wird dabei einerseits komprimiert und andererseits umgebogen, so dass die Zunge 32 aufgrund der konischen Gestalt des Prothesenschaftes 40 und der Einführbewegung in Richtung auf das proximale Ende des Linersystems 1 umgebogen wird. Die in dem Volumen eingeschlossene, komprimierte Luft kann bei zunehmendem Druck während des Einsteigens in den Prothesenschaft 40 an der Außenseite der Dichtlippe und des Außenliners 20 entweichen.

Um einerseits zu verhindern, dass bei dem seitlichen Entweichen zwischen der Prothesenschaftinnenwand 41 und dem Außenliner 20 unangenehme Geräusche entstehen und andererseits ein Aussteigen aus dem Prothesenschaft 40 zu ermöglichen, ist in der Prothesenschaftwand ein Ventil 50 vorgesehen, das zum Entlüften und Belüften des Zwischenraumes zwischen dem Außenliner 20 und der Innenseite 41 des Prothesenschaftes 40 dient. Bei einem Einsteigen wird automatisch Luft aus dem abgeschlossenen Volumen zwischen dem Prothesenschaft 40 und der Außenseite des Linersystems 1 herausgedrückt. Möchte der Patient aus dem Prothesenschaft 40 aussteigen, also den Stumpf zusammen mit dem Linersystem 1 in proximaler Richtung herausziehen, wird das Ventil 50 beispielsweise manuell betätigt, so dass Luft in den Zwischenraum einströmen kann, so dass zum Aussteigen oder Herausziehen des Linersystems 1 aus dem Prothesenschaft nur noch eventuell vorhandene Haftkräfte zwischen dem Außenliner 20 und der Innenwand 41 des Prothesenschaftes und elastische Rückstellkräfte über das Dichtelement 30 zu überwinden sind.

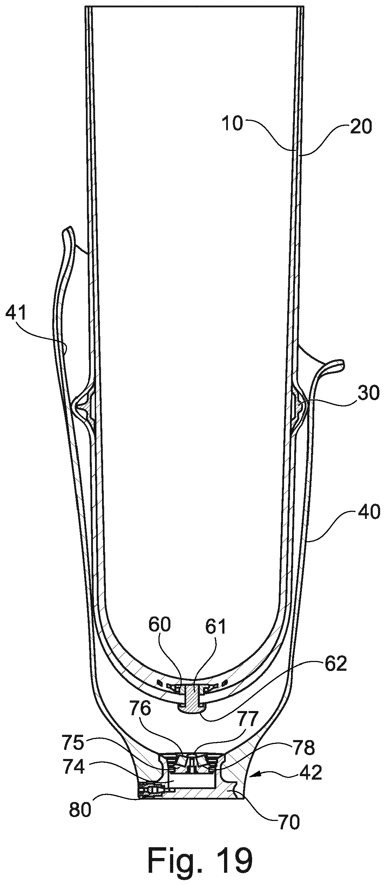

In den Figuren 19 bis 21 ist schematisch eine weitere Variante der Erfindung dargestellt. Der grundsätzliche Aufbau des Prothesenlinersystems 1 entspricht dem der Figur 18, jedoch ist in dem Innenliner 10 eine Halterung 60 eingearbeitet, die sich in dem distalen Endbereich 11 des Innenliners 10 befindet. Die Halterung 60 ist bevorzugt zentral in dem Innenliner 10 angeordnet, insbesondere einlaminiert oder eingegossen. Die Halterung 60 kann sich bis zur Außenwand 16 des Innenliners 10 erstrecken. Innerhalb der Halterung 60 ist ein Zapfen 61 angeordnet, beispielsweise eingesetzt, eingeschraubt oder eingepresst. Der Zapfen 61 ragt durch den Außenliner 20 hindurch und weist einen distalen Kopf 62 auf, der an der Außenseite 26 des Außenliners 20 anliegt. Der Kopf 62 kann als separate Komponente an dem Zapfen 61 angeordnet sein. Der Kopf 62 kann aus einem ferromagnetischen Material bestehen, eine ferromagnetische Beschichtung aufweisen oder mit Magneten ausgestattet oder als Magnet ausgebildet sein.

Wir das Linersystem 1 in den Prothesenschaft 40 eingeführt, wie es in der Figur 19 dargestellt ist, legt sich der Außenumfang im Bereich des Dichtelementes 30 an die Innenwand 41 des Prothesenschaftes 40 an. Zeichnerisch ist dies dadurch dargestellt, dass die ursprüngliche Außenkontur des Linersystems 1 über die Innenkontur des Prothesenschaftes 40 hinausragt. Bei einer tatsächlichen Benutzung würde sich die Dichtlippe 30 verformen und damit auch der Außenliner 20 und sich an die Innenkontur an der Innenoberfläche 41 des Prothesenschaftes 40 anpassen und ein geschlossenes Volumen zwischen der Innenwand 41 des Prothesenschaftes 40 und dem Außenliner 20 abdichten.

Innerhalb des Prothesenliners 40 ist im distalen Endbereich 42 eine Verdickung oder Vorsprung ausgebildet, in der ein Einsatz 70 eingearbeitet oder festgelegt ist, an dem in distaler Richtung weitere Prothesenkomponenten befestigt werden können. In dem Einsatz 70 ist eine Ausnehmung 74 ausgearbeitet, in der ein Kolben 75 verschieblich in distal-proximal-Richtung gelagert ist. Die Ausnehmung 74 kann bevorzugt zylindrisch ausgebildet sein. In dem korrespondierend geformten Kolben 75 sind in dem dargestellten Ausführungsbeispiel mehrere Magnete 76 angeordnet, um eine kraftschlüssige Kopplung mit dem Kopf 62 an dem Prothesenlinersystem 1 zu gewährleisten. Der Kolben 75 und die Ausnehmung 74 bilden zusammen mit dem Einsatz eine Pumpkammer, die in strömungstechnischer Verbindung mit einem Auslassventil 80 steht. Innerhalb des Kolbens 75 kann ein mit einem Ventil 77 versehener Strömungsdurchgang 78 vorgesehen sein, um darüber im Rahmen einer Pumpbewegung des Kolbens 75 Luft aus dem Volumen zwischen dem Prothesenschaft 40 und dem Linersystem 1 abzusaugen und durch das Auslassventil 80 in die Umgebung abzutransportieren.

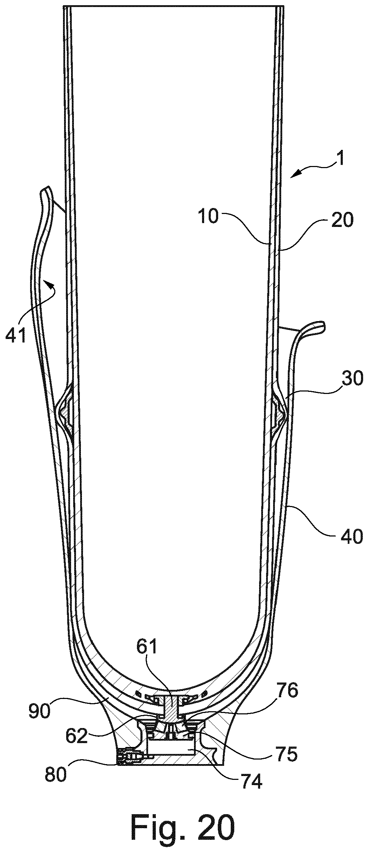

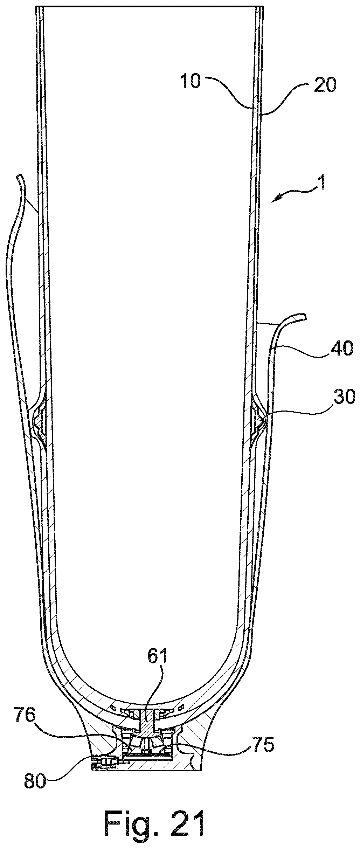

Figur 20 zeigt die Ausgestaltung gemäß Figur 19 in einem montierten Zustand, bei dem der Zapfen 61 mit dem Kopf 62 in Kontakt mit dem Kolben 75 und den daran angeordneten Magneten 76 steht. Über die Magnete 76 und den gegebenenfalls magnetischen, zumindest ferromagnetischen Kopf 62 wird eine kraftschlüssige Verbindung zwischen dem Kolben 75 und dem Prothesenlinersystem 1 hergestellt. Das Prothesenlinersystem 1 ist weiter in distaler Richtung in den Prothesenschaft 40 eingeführt, die Dichtung 30 ist an der Innenseite 41 des Prothesenschaftes 40 entlangführt und ist aufgrund der fortschreitenden Annäherung der Innenwand 41 an die Außenkontur des nicht durch das Dichtelement 30 aufgeweiteten Außenliners 20 stärker verformt. Dies ist zeichnerisch durch die vergrößerte Überdeckung der unverformt dargestellten Komponenten aus Prothesenschaft 40 und Prothesenlinersystem 1 dargestellt. Ebenfalls dargestellt ist das geschlossene Volumen 90, das sich zwischen der dichtenden Anlage des Dichtelementes 30 und des Außenliners 20 an der Innenwand 41 des Prothesenschaftes im proximalem Bereich und der geschlossenen Ausgestaltung des Prothesenschaftes 40 im distalen Bereich ausbildet. Das Pumpvolumen, das im dargestellten Zustand maximal ist, da sich der Kolben 75 in der Proximalstellung befindet, wird durch weiteres Einführen des Prothesenlinersystems 1 in Distalrichtung verringert, bis der Außenliner 20 vollständig an dem distalen inneren Ende des Prothesenschaftes 40 anliegt. Dieser Zustand ist in der Figur 21 gezeigt. Das in dem Pumpvolumen in der Ausnehmung 74 eingeschlossene Volumen wird durch das Auslassventil 80 herausgedrückt. Eine solche Pumpbewegung findet beispielsweise während der Standphase beim Gehen oder während des Stehens statt. Wird der Prothesenschaft 40 entlastet, also in Distalrichtung relativ zu dem Prothesenlinersystem 1 bewegt, beispielsweise während einer Schwungphase oder einer Entlastungsphase, wird aufgrund der kraftschlüssigen Verbindung zwischen dem Zapfen 61 und dem Kolben 75 der Kolben 75 in Proximalrichtung verlagert. Über das Ventil 77 kann Luft aus dem abgeschlossenen Volumen 90, das beispielsweise über eine Leckage hineingelangt ist, abgesaugt und bei der nächsten Belastung durch das Auslassventil 80 ausgestoßen werden. Die Ventile 77, 80 können auch als Umschaltventile ausgebildet sein, um eine Belüftung des Volumens 90 zum Aussteigen zu ermöglichen.

Neben einer dargestellten magnetischen Kopplung zwischen Kolben 75 und Prothesenlinersystem 1 kann dies auch auf eine andere Art und Weise kraftschlüssig erfolgen; grundsätzlich sind auch formschlüssige Kopplungen vorgesehen, beispielsweise über einen federnden Rastverschluss, Klettverschlüsse, einen Bajonett-Mechanismus oder ähnliches.

An das Auslassventil 80 kann eine aktive Pumpe angeschlossen werden, um Unterdruck in dem von dem Kolben 75 und der Ausnehmung 74 gebildeten Pumpvolumen zu erzeugen, so dass eine zusätzliche Sicherung des Prothesenlinersystems 1 innerhalb des Prothesenschaftes 40 gewährleistet werden kann.

## Patentansprüche

1. Linersystem (1), zur Anlage an einer Gliedmaße oder einem Gliedmaßenstumpf, mit einem Innenliner (10), der eine der Gliedmaße oder dem Gliedmaßenstumpf zugewandte Innenseite (15) und eine der Gliedmaße oder dem Gliedmaßenstumpf abgewandten Außenseite (16) aufweist, mit einem Außenliner (20), der eine dem Innenliner (10) zugewandte Innenseite (25) und einem dem Innenliner (10) abgewandte Außenseite (26) aufweist und zur Anlage über dem Innenliner (10) ausgebildet ist, mit einem separat von dem Innenliner (10) und dem Außenliner (20) ausgebildeten Dichtelement (30) zur Anordnung zwischen der Außenseite (16) des Innenliners (10) und der Innenseite (25) des Außenliners (20) zur Vergrößerung der Materialdicke im Bereich des Dichtelementes (30), **dadurch gekennzeichnet, dass** das Dichtelement (30) ringförmig oder bandartig ausgebildet ist.

2. Linersystem nach Anspruch 1, **dadurch gekennzeichnet, dass** das Dichtelement (30) bandartig mit zwei freien Enden (32, 33) ausgebildet und mit zumindest einem Befestigungselement (35) zur Festlegung der freier Enden (32, 33) aneinander versehen ist.

3. Linersystem nach Anspruch 2, **dadurch gekennzeichnet, dass** das Befestigungselement (35) an dem Dichtelement (30) ausgebildet oder befestigt ist.

4. Linersystem nach einem der voranstehenden Ansprüche, **dadurch gekennzeichnet, dass** der Querschnitt des Dichtelementes (30) massiv oder als geschlossener oder offener Hohlquerschnitt ausgebildet ist.

5. Linersystem nach Anspruch 4, **dadurch gekennzeichnet, dass** das Dichtelement (30) aus einem Schaumwerkstoff gebildet oder mit einem Schaumwerkstoff oder einem Textil versehen oder gefüllt ist.

6. Linersystem nach einem der voranstehenden Ansprüche, **dadurch gekennzeichnet, dass** der Querschnitt des Dichtelemente (30) rund, dreieckig, viereckig oder polygonal oder mit einer flachen Anlagefläche (31) ausgebildet ist.

7. Linersystem nach einem der voranstehenden Ansprüche, **dadurch gekennzeichnet, dass** das Dichtelement (30) elastisch ausgebildet ist oder aus einem elastischem Material besteht.

8. Linersystem nach einem der voranstehenden Ansprüche, **dadurch gekennzeichnet, dass** das Dichtelement (30) auf der Außenseite (15) des Innenliners (10) und/oder der Innenseite (25) des Außenliners (20) haftet.

9. Linersystem nach einem der voranstehenden Ansprüche, **dadurch gekennzeichnet, dass** der Innenliner (10) und/oder der Außenliner (20) einen geschlossen, distalen Endbereich (11, 22) aufweisen.

10. Linersystem nach einem der voranstehenden Ansprüche, **dadurch gekennzeichnet, dass** der Innenliner (10) und der Außenliner (20) aneinander festgelegt sind.

11. Linersystem nach Anspruch 10, **dadurch gekennzeichnet, dass** der Innenliner (10) und der Außenliner (20) in ihren distalen Endbereichen (11, 22) formschlüssig oder stoffschlüssig miteinander verbunden sind und eine Verbindungszone (21) ausbilden.

12. Linersystem nach einem der voranstehenden Ansprüche, **dadurch gekennzeichnet, dass** der Innenliner (10) und/oder der Außenliner (20) einen geschlossenen Querschnitt aufweisen.

13. Linersystem nach einem der voranstehenden Ansprüche, **dadurch gekennzeichnet, dass** der Außenliner (20) oder der Innenliner (10) und der Außenliner (20) elastisch oder aus einem Elastomer ausgebildet sind.

14. Linersystem nach einem der voranstehenden Ansprüche, **dadurch gekennzeichnet, dass** der Innenliner (10) und der Außenliner (20) aus dem gleichen Material bestehen.

15. Linersystem nach Anspruch 11, **dadurch gekennzeichnet, dass** sich die Verbindungszone (21) in Proximalrichtung nicht weiter als bis zu einem ersten distalen Drittel des Außenliners (20) erstreckt.

16. Linersystem nach einem der voranstehenden Ansprüche, **dadurch gekennzeichnet, dass** auf dem Innenliner (10) Markierungen (13) zur Ausrichtung des Dichtelementes (30) angeordnet sind.

17. Linersystem nach einem der voranstehenden Ansprüche, **dadurch gekennzeichnet, dass** der Innenliner (10) zusammen mit dem Außenliner (20) eine glattwandige Innenoberfläche ausbildet.

18. Linersystem nach einem der voranstehenden Ansprüche, **dadurch gekennzeichnet, dass** der Außenliner (20) kürzbar ausgebildet ist.

19. Verfahren zum Anlegen eines Linersystems nach einem der voranstehenden Ansprüche, mit den Schritten
- Anlegen des Innenliners (10) um eine Gliedmaße oder einen Gliedmaßenstumpf, gefolgt von
- Auflegen des ringförmig oder bandartig ausgebildeten, separaten Dichtelements (30) auf eine Außenseite (16) des Innenliners (10), gefolgt von
- Anlegen des Außenliners (30) auf die Außenseite (16) des Innenliners (10) und Überdecken des Dichtelementes (30) mit dem Außenliner (20).

20. Verfahren nach Anspruch 19, **dadurch gekennzeichnet, dass** der Außenliner (20) bei dem Anlegen oder nach dem Anlegen des Innenliners (10) zumindest teilweise über der Außenseite (16) des Innenliners (10) angeordnet wird, dass anschließend ein proximales Ende (27) des Außenliners (10) in distaler Richtung heruntergezogen wird, bevor das Dichtelement (30) aufgelegt wird, und dass anschließend das heruntergezogene proximale Ende (27) des Außenliners (20) über das auf den Innenliner (10) aufgelegte Dichtelement (30) bewegt wird.

21. Verfahren nach Anspruch 19 oder 20, **dadurch gekennzeichnet, dass** das Dichtelement (30) schräg oder senkrecht zu der Längserstreckung des Innenliners (10) auf der Außenseite (16) des Innenliners (10) aufgelegt wird.

22. Verfahren nach einem der Ansprüche 19 bis 21, **dadurch gekennzeichnet, dass** das Dichtelement (30) nach oder bei dem Auflegen auf die Außenseite (16) des Innenliners (10) an dem Innenliner (10) fixiert wird.

## Claims

1. A liner system (1) for applying to a limb or a limb stump, with an inner liner (10) comprising an inner side (15) facing the limb or limb stump and an outer side (16) facing away from the limb or limb stump, and an outer liner (20) that comprises an inner side (25) facing the inner liner (10) and an outer side (26) facing away from the inner liner (10) and that is designed to be applied over the inner liner (10), with a sealing element (30), which is designed to be separate from the inner liner (10) and the outer liner (20), for arranging between the outer side (16) of the inner liner (10) and the inner side (25) of the outer liner (20) to increase the material thickness, **characterized in that** the sealing element (30) is designed to be ring-shaped or belt-shaped.

2. The liner system according to claim 1, **characterized in that** the sealing element (30) is designed to be belt-shaped with two free ends (32, 33) and features at least one fixing element (35) for attaching the free ends (32, 33) to one another.

3. The liner system according to claim 2, **characterized in that** the fixing element (35) is configured on or fixed to the sealing element (30).

4. The liner system according to one of the above claims, **characterized in that** the cross-section of the sealing element (30) is designed to be solid or as a closed or open hollow cross-section.

5. The liner system according to claim 4, **characterized in that** the sealing element (30) is made of a foam material or features or is filled with a foam material or a textile.

6. The liner system according to one of the above claims, **characterized in that** the cross-section of the sealing element (30) is designed to be round, triangular, quadrangular or polygonal, or with a flat contact surface (31).

7. The liner system according to one of the above claims, **characterized in that** the sealing element (30) is designed to be elastic or is made of an elastic material.

8. The liner system according to one of the above claims, **characterized in that** the sealing element (30) sticks to the outer side (15) of the inner liner (10) and/or to the inner side (25) of the outer liner (20).

9. The liner system according to one of the above claims, **characterized in that** the inner liner (10) and/or the outer liner (20) comprise a closed distal end region (11, 22).

10. The liner system according to one of the above claims, **characterized in that** the inner liner (10) and the outer liner (20) are attached to one another.

11. The liner system according to claim 10, **characterized in that** the inner liner (10) and the outer liner (20) are connected to one another in their distal end regions (11, 22) in either a positive-locking or bonded manner, and form a connection zone (21).

12. The liner system according to one of the above claims, **characterized in that** the inner liner (10) and/or the outer liner (20) comprises a closed cross-section.

13. The liner system according to one of the above claims, **characterized in that** the outer liner (20) or the inner liner (10) and the outer liner (20) are designed to be elastic or made of an elastomer.

14. The liner system according to one of the above claims, **characterized in that** the inner liner (10) and the outer liner (20) are made of the same material.

15. The liner system according to claim 11, **characterized in that** the connection zone (21) does not extend beyond up to a first distal third of the outer liner (20) in the proximal direction.

16. The liner system according to one of the above claims, **characterized in that** markers (13) are arranged on the inner liner (10) for the purpose of aligning the sealing element (30).

17. The liner system according to one of the above claims, **characterized in that**, along with the outer liner (20), the inner liner (10) forms a smooth-walled inner surface.

18. The liner system according to one of the above claims, **characterized in that** the outer liner (20) is designed such that it can be shortened.

19. A method for applying a liner system according to one of the above claim, featuring the steps
- application of the inner liner (10) around a limb or a limb stump, followed by
- application of the ring-shaped or belt-shaped, separate sealing element (30) on an outer side (16) of the inner liner (10), followed by
- application of the outer liner (30) on the outer side (16) of the inner liner (10) and covering of the sealing element (30) with outer liner (20).

20. The method according to claim 19, **characterized in that**, upon or following the application of the inner liner (10), the outer liner (20) is arranged at least partially over the outer side (16) of the inner liner (10); that a proximal end (27) of the outer liner (10) is subsequently pulled down in the distal direction before the application of the sealing element (30); and that the pulled-down distal end (27) of the outer liner (20) is subsequently moved over the sealing element (30) applied on the inner liner (10).

21. The method according to claim 19 or 20, **characterized in that** the sealing element (30) is applied on the outer side (16) of the inner liner (10) transversely or perpendicular to the longitudinal direction of the inner liner (10).

22. The method according to one of the claims 19 to 21, **characterized in that** the sealing element (30) is fixed to the inner liner (10) following or upon the application to the outer side (16) of the inner liner (10).

## Revendications

1. Système de doublures (1) destiné à être appliqué sur un membre ou un moignon de membre, comprenant une doublure intérieure (10) ayant un côté intérieur (15) tourné vers le membre ou le moignon de membre et un côté extérieur (16) détourné du membre ou du moignon de membre, une doublure extérieure (20) ayant un côté intérieur (25) tourné vers la doublure intérieure (10) et un côté extérieur (26) détourné de la doublure intérieure (10) et réalisée pour être appliquée par-dessus la doublure intérieure (10), un élément d'étanchéité (30) réalisé séparément de la doublure intérieure (10) et de la doublure extérieure (20) et destiné à être placé entre le côté extérieur (16) de la doublure intérieure (10) et le côté intérieur (25) de la doublure extérieure (20) afin d'augmenter l'épaisseur du matériau dans la zone de l'élément d'étanchéité (30),
**caractérisé en ce que** l'élément d'étanchéité (30) est réalisé en forme annulaire ou en forme de bande.

2. Système de doublures selon la revendication 1,
**caractérisé en ce que** l'élément d'étanchéité (30) est réalisé en forme de bande ayant deux extrémités libres (32, 33) et est pourvu d'au moins un élément de fixation (35) pour immobiliser les extrémités libres (32, 33) l'une à l'autre.

3. Système de doublures selon la revendication 2,
**caractérisé en ce que** l'élément de fixation (35) est réalisé ou fixé à l'élément d'étanchéité (30).

4. Système de doublures selon l'une des revendications précédentes,
**caractérisé en ce que** la section transversale de l'élément d'étanchéité (30) est pleine ou réalisée sous forme de section transversale creuse fermée ou ouverte.

5. Système de doublures selon la revendication 4,
**caractérisé en ce que** l'élément d'étanchéité (30) est réalisé en un matériau en mousse ou est pourvu ou rempli d'un matériau en mousse ou d'un textile.

6. Système de doublures selon l'une des revendications précédentes,
**caractérisé en ce que** la section transversale de l'élément d'étanchéité (30) est ronde, triangulaire, quadrangulaire ou polygonale ou est réalisée avec une surface d'appui plate (31).

7. Système de doublures selon l'une des revendications précédentes,
**caractérisé en ce que** l'élément d'étanchéité (30) est réalisé de façon élastique ou est constitué d'un matériau élastique.

8. Système de doublures selon l'une des revendications précédentes,
**caractérisé en ce que** l'élément d'étanchéité (30) adhère au côté extérieur (15) de la doublure intérieure (10) et/ou au côté intérieur (25) de la doublure extérieure (20).

9. Système de doublures selon l'une des revendications précédentes,
**caractérisé en ce que** la doublure intérieure (10) et/ou la doublure extérieure (20) présente(nt) une zone d'extrémité distale fermée (11, 22).

10. Système de doublures selon l'une des revendications précédentes,
**caractérisé en ce que** la doublure intérieure (10) et la doublure extérieure (20) sont immobilisées l'une à l'autre.

11. Système de doublures selon la revendication 10,
**caractérisé en ce que** la doublure intérieure (10) et la doublure extérieure (20) sont reliées l'une à l'autre par coopération de forme ou de matière dans leurs zones d'extrémité distales (11, 22) et forment une zone de liaison (21).

12. Système de doublures selon l'une des revendications précédentes,
**caractérisé en ce que** la doublure intérieure (10) et/ou la doublure extérieure (20) présente(nt) une section transversale fermée.

13. Système de doublures selon l'une des revendications précédentes,
**caractérisé en ce que** la doublure extérieure (20) ou la doublure intérieure (10) et la doublure extérieure (20) sont réalisées de façon élastique ou en un élastomère.

14. Système de doublures selon l'une des revendications précédentes,
**caractérisé en ce que** la doublure intérieure (10) et la doublure extérieure (20) sont réalisées en le même matériau.

15. Système de doublures selon la revendication 11,
**caractérisé en ce que** la zone de liaison (21) s'étend dans la direction proximale pas plus loin que jusqu'à un premier tiers distal de la doublure extérieure (20).

16. Système de doublures selon l'une des revendications précédentes,
**caractérisé en ce que** des repères (13) pour l'alignement de l'élément d'étanchéité (30) sont disposés sur la doublure intérieure (10).

17. Système de doublures selon l'une des revendications précédentes,
**caractérisé en ce que** la doublure intérieure (10) forme conjointement avec la doublure extérieure (20) une surface intérieure à paroi lisse.

18. Système de doublures selon l'une des revendications précédentes,
**caractérisé en ce que** la doublure extérieure (20) est réalisée de manière à pouvoir être raccourcie.

19. Procédé d'application d'un système de doublures selon l'une des revendications précédentes, comprenant les étapes consistant à :
- appliquer une doublure intérieure (10) autour d'un membre ou d'un moignon de membre, puis
- poser l'élément d'étanchéité (30) séparé, réalisé en forme annulaire ou en forme de bande, sur un côté extérieur (16) de la doublure intérieure (10), puis
- appliquer la doublure extérieure (30) sur le côté extérieur (16) de la doublure intérieure (10), et recouvrir l'élément d'étanchéité (30) avec la doublure extérieure (20).

20. Procédé selon la revendication 19,
**caractérisé en ce que** la doublure extérieure (20) est placée au moins partiellement sur le côté extérieur (16) de la doublure intérieure (10) pendant l'application ou après l'application de la doublure intérieure (10),
**en ce qu'**ensuite une extrémité proximale (27) de la doublure extérieure (10) est tirée vers le bas dans la direction distale avant que l'élément d'étanchéité (30) soit posé, et
**en ce qu'**ensuite l'extrémité proximale (27), tirée vers le bas, de la doublure extérieure (20) est déplacée par-dessus l'élément d'étanchéité (30) posé sur la doublure intérieure (10).

21. Procédé selon la revendication 19 ou 20,
**caractérisé en ce que** l'élément d'étanchéité (30) est posé sur le côté extérieur (16) de la doublure intérieure (10) en oblique ou perpendiculairement à l'extension longitudinale de la doublure intérieure (10).

22. Procédé selon l'une des revendications 19 à 21,
**caractérisé en ce que** l'élément d'étanchéité (30) est fixé à la doublure intérieure (10) après ou pendant l'application sur le côté extérieur (16) de la doublure intérieure (10).
